# EUROPEAN PATENT SPECIFICATION

(11) **EP 2 049 529 B1**
(45) Date of publication and mention of the grant of the patent: **11.08.2010**
(21) Application number: 07796852.7
(22) Date of filing: 13.07.2007
(51) Int. Cl.: C07D 403/14, C07D 413/14, C07D 417/14, A61K 31/551, A61P 25/20

(54) **SUBSTITUTED DIAZEPAN OREXIN RECEPTOR ANTAGONISTS**
SUBSTITUIERTE DIAZEPANE ALS ANTAGONISTEN AN OREXINREZEPTOREN
DIAZÉPANS SUBSTITUÉS ANTAGONISTES DU RÉCEPTEUR DE L'ORÉXINE

(30) Priority: 14.07.2006 US 830983 P
(43) Date of publication of application: 22.04.2009
(73) Proprietor: Merck Sharp & Dohme Corp., Rahway, NJ 07065 (US)
(72) Inventor: COX, Christopher D., Rahway, New Jersey 07065-0907 (US); BRESLIN, Michael J., Rahway, New Jersey 07065-0907 (US); COLEMAN, Paul J., Rahway, New Jersey 07065-0907 (US)
(74) Representative: Buchan, Gavin MacNicol
(86) International application number: PCT/US2007/016038
(87) International publication number: WO 2008/008518

(56) References cited:
- EP-A- 0 385 237
- CAI J ET AL: "Antagonists of the orexin receptors" EXPERT OPINION ON THERAPEUTIC PATENTS 2006 UNITED KINGDOM, vol. 16, no. 5, May 2006 (2006-05), pages 631-646, XP002458093 ISSN: 1354-3776

## Description

### BACKGROUND OF THE INVENTION

The orexins (hypocretins) comprise two neuropeptides produced in the hypothalamus: the orexin A (OX-A) (a 33 amino acid peptide) and the orexin B (OX-B) (a 28 amino acid peptide) (Sakurai T. et al., Cell, 1998, 92, 573-585). Orexins are found to stimulate food consumption in rats suggesting a physiological role for these peptides as mediators in the central feedback mechanism that regulates feeding behaviour (Sakurai T. et al., Cell, 1998, 92, 573-585). Orexins also regulate states of sleep and wakefulness opening potentially novel therapeutic approaches for narcoleptic or insomniac patients (Chemelli R.M. et al., Cell, 1999, 98, 437-451). Two orexin receptors have been cloned and characterized in mammals. They belong to the super family of G-protein coupled receptors (Sakurai T. et al., Cell, 1998, 92, 573-585): the orexin-1 receptor (OX or OX1R) is selective for OX-A and the orexin-2 receptor (OX2 or OX2R) is capable to bind OX-A as well as OX-B. The physiological actions in which orexins are presumed to participate are thought to be expressed via one or both of OX 1 receptor and OX 2 receptor as the two subtypes of orexin receptors.

Orexin receptors are found in the mammalian brain and may have numerous implications in pathologies such as depression; anxiety; addictions; obsessive compulsive disorder; affective neurosis; depressive neurosis; anxiety neurosis; dysthymic disorder; behaviour disorder; mood disorder; sexual dysfunction; psychosexual dysfunction; sex disorder; schizophrenia; manic depression; delirium; dementia; severe mental retardation and dyskinesias such as Huntington's disease and Tourette syndrome; eating disorders such as anorexia, bulimia, cachexia, and obesity; cardiovascular diseases; diabetes; appetite/taste disorders; emesis, vomiting, nausea; asthma; cancer; Parkinson's disease; Cushing's syndrome/disease; basophile adenoma; prolactinoma; hyperprolactinemia; hypophysis tumour/adenoma; hypothalamic diseases; inflammatory bowel disease; gastric diskinesia; gastric ulcers; Froehlich's syndrome; adrenohypophysis disease; hypophysis disease; adrenohypophysis hypofunction; adrenohypophysis hyperfunction; hypothalamic hypogonadism; Kallman's syndrome (anosmia, hyposmia); functional or psychogenic amenorrhea; hypopituitarism; hypothalamic hypothyroidism; hypothalamic- adrenal dysfunction; idiopathic hyperprolactinemia; hypothalamic disorders of growth hormone deficiency; idiopathic growth deficiency; dwarfism; gigantism; acromegaly; disturbed biological and circadian rhythms; sleep disturbances associated with diseases such as neurological disorders, neuropathic pain and restless leg syndrome; heart and lung diseases, acute and congestive heart failure; hypotension; hypertension; urinary retention; osteoporosis; angina pectoris; myocardinal infarction; ischemic or haemorrhagic stroke; subarachnoid haemorrhage; ulcers; allergies; benign prostatic hypertrophy; chronic renal failure; renal disease; impaired glucose tolerance; migraine; hyperalgesia; pain; enhanced or exaggerated sensitivity to pain such as hyperalgesia, causalgia, and allodynia; acute pain; bum pain; atypical facial pain; neuropathic pain; back pain; complex regional pain syndrome I and II; arthritic pain; sports injury pain; pain related to infection e.g. HIV, post-chemotherapy pain; post-stroke pain; post-operative pain; neuralgia; emesis, nausea, vomiting; conditions associated with visceral pain such as irritable bowel syndrome, and angina; migraine; urinary bladder incontinence e.g. urge incontinence; tolerance to narcotics or withdrawal from narcotics; sleep disorders; sleep apnea; narcolepsy; insomnia; parasomnia; jet lag syndrome; and neurodegenerative disorders including nosological entities such as disinhibition-dementia-parkinsonism-amyotrophy complex; pallido-ponto-nigral degeneration; epilepsy; seizure disorders and other diseases related to general orexin system dysfunction.

Certain orexin receptor antagonists are disclosed in PCT patent publications WO 99/09024, WO 99/58533, WO 00/47576, WO 00/47577, WO 00/47580, WO 01/68609, WO 01/85693, WO 2002/051232, WO 2002/051838, WO 2003/002559, WO 2003/002561, WO 2003/032991, WO 2003/037847, WO 2003/041711, WO 2003/051872, WO 2003/051873, WO 2004/004733, WO 2004/033418, WO 2004/083218, WO 2004/085403, WO 2005/060959, WO2005/118548 2-Amino-methylpiperidine derivatives (WO 01/96302), 3-aminomethyl morpholine derivatives (WO 02/44172) and N-aroyl cyclic amines (WO 02/090355, WO 02/089800 and WO 03/051368) are disclosed as orexin receptor antagonists.

### SUMMARY OF THE INVENTION

The present invention is directed to diazepan compounds which are antagonists of orexin receptors, and which are useful in the treatment or prevention of neurological and psychiatric disorders and diseases in which orexin receptors are involved. The invention is also directed to pharmaceutical compositions comprising these compounds and the use of these compounds and compositions in the prevention or treatment of such diseases in which orexin receptors are involved.

### DETAILED DESCRIPTION OF THE INVENTION

The present invention is directed to compounds of the formula I: 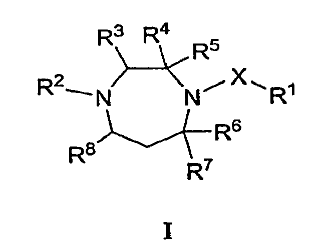 wherein:
X is selected from -SO₂-, -(C=O)-, and -(C=S)-;
R¹ is selected from the group consisting of:
   (1) phenyl, where the phenyl is substituted with R^{1a}, R^{1b} and R^{1c}, and
   (2) napthyl, where the napthyl is substituted with R^{1a}, R^{1b} and R^{1c};
   (3) heteroaryl, where the heteroaryl is substituted with R^{1a}, R^{1b} and R^{1c};
R² is heteroaryl, where the heteroaryl is substituted with R^{2a}, R^{2b} and R^{2c};
R^{1a}, R^{1b}, R^{1c}, R^{2a}, R^{2b} and R^{2c} are independently selected from the group consisting of:
   (1) hydrogen,
   (2) halogen,
   (3) hydroxyl,
   (4) -(C=O)ₘ-Oₙ-C₁₋₆alkyl, where m is 0 or 1, n is 0 or 1 (wherein if m is 0 or n is 0, a bond is present) and where the alkyl is unsubstituted or substituted with one or more substituents selected from R¹³,
   (5) -(C=O)ₘ-Oₙ-C₃-₆cycloalkyl, where the cycloalkyl is unsubstituted or substituted with one or more substituents selected from R13,
   (6) -(C=O)ₘ-C₂₋₄alkenyl, where the alkenyl is unsubstituted or substituted with one or more substituents selected from R¹³,
   (7) -(C=O)ₘ-C₂-₄alkynyl, where the alkenyl is unsubstituted or substituted with one or more substituents selected from R¹³,
   (8) -(C=O)ₘ-Oₙ-phenyl or -(C=O)ₘ-Oₙ-napthyl, where the phenyl or napthyl is unsubstituted or substituted with one or more substituents selected from R¹³,
   (9) -(C=O)ₘ-Oₙ-heterocycle, where the heterocycle is unsubstituted or substituted with one or more substituents selected from R13,
   (10) -(C=O)ₘ-NR¹⁰R¹¹, wherein R¹⁰ and R¹¹ are independently selected from the group consisting of:
      (a) hydrogen,
      (b) C₁₋₆alkyl, which is unsubstituted or substituted with one or more substituents selected from R¹³,
      (c) C₃₋₆alkenyl, which is unsubstituted or substituted with one or more substituents selected from R¹³,
      (d) cycloalkyl, which is unsubstituted or substituted with one or more substituents selected from R¹³,
      (e) phenyl, which is unsubstituted or substituted with one or more substituents selected from R¹³, and
      (f) heterocycle, which is unsubstituted or substituted with one or more substituents selected from R¹³,
   (11) -S(O)₂-NR¹⁰R¹¹,
   (12) -S(O)_{q}-R¹², where q is 0, 1 or 2 and where R¹² is selected from the definitions of R¹⁰ and R¹¹,
   (13) -CO₂H,
   (14) -CN,
   (15) -NO₂, and
   (16) -B(OH)₂;
R³, R⁴, R⁵, R⁶, R⁷ and R⁸ are independently selected from the group consisting of:
   (1) hydrogen,
   (2) -C₁₋₆alkyl, where the alkyl is unsubstituted or substituted with one or more substituents selected from R¹³,
   (3) -O-C₁₋₆alkyl, where the alkyl is unsubstituted or substituted with one or more substituents selected from R¹³, and
   (4) -phenyl, where the phenyl is unsubstituted or substituted with one or more substituents selected from R¹³,
      or R⁴ and R⁵, or R⁶ and R⁷ are taken together to form a C₃₋₆cycloalkyl, where the cycloalkyl is unsubstituted or substituted with one or more substituents selected from R¹⁴,
      with the proviso that at least one of R³, R⁴, R⁵, R⁶, R⁷ or R⁸ is other than hydrogen;
R¹³ is selected from the group consisting of:
   (1) halogen,
   (2) hydroxyl,
   (3) -(C=O)ₘ-Oₙ-C₁-₆alkyl, where the alkyl is unsubstituted or substituted with one or more substituents selected from R¹⁴,
   (4) -Oₙ-(C₁₋₃)perfluoroalkyl,
   (5) -(C=O)ₘ-Oₙ-C₃₋₆cycloalkyl, where the cycloalkyl is unsubstituted or substituted with one or more substituents selected from R¹⁴,
   (6) -(C=O)ₘ-C₂₋₄alkenyl, where the alkenyl is unsubstituted or substituted with one or more substituents selected from R¹⁴,
   (7) -(C=O)ₘ-Oₙ-phenyl or -(C=O)ₘ-Oₙ-napthyl, where the phenyl or napthyl is unsubstituted or substituted with one or more substituents selected from R¹⁴,
   (8) -(C=O)ₘ-Oₙ-heterocycle, where the heterocycle is unsubstituted or substituted with one or more substituents selected from R¹⁴,
   (9) -(C=O)ₘ-NR¹⁰R¹¹,
   (10) -S(O)₂-NR¹⁰R¹¹,
   (11) -S(O)_{q}-R¹²,
   (12) -CO₂H,
   (13) -CN, and
   (14) -N02;
R14 is selected from the group consisting of:
   (1) hydroxyl,
   (2) halogen,
   (3) C₁₋₆alkyl,
   (4) -C₃₋₆cycloalkyl,
   (5) -O-C₁₋₆alkyl,
   (6) -O(C=O)-C₁₋₆alkyl,
   (7) -NH-C₁₋₆alkyl,
   (8) phenyl,
   (9) heterocycle,
   (10) -CO₂H, and
   (11) -CN;
or a pharmaceutically acceptable salt thereof.

An embodiment of the present invention includes compounds of the formula Ia: 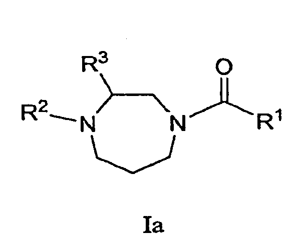 wherein R¹, R² and R³ are defined herein; or a pharmaceutically acceptable salt thereof.

An embodiment of the present invention includes compounds of the formula Ib: 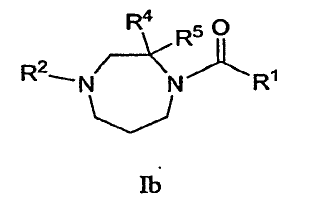 wherein R¹, R², R⁴ and R⁵ are defined herein; or a pharmaceutically acceptable salt thereof.

An embodiment of the present invention includes compounds of the formula Ic: 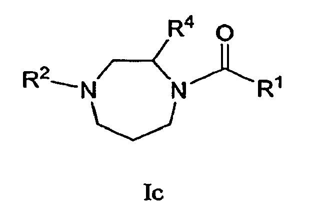 wherein R¹, R² and R⁴ are defined herein; or a pharmaceutically acceptable salt thereof.

An embodiment of the present invention includes compounds of the formula Ic': 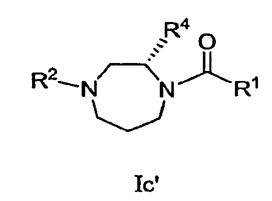 wherein R¹, R² and R⁴ are defined herein; or a pharmaceutically acceptable salt thereof.

An embodiment of the present invention includes compounds of the formula Id: 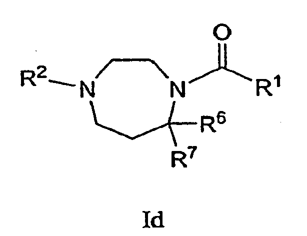 wherein R¹, R², R⁶ and R⁷ are defined herein; or a pharmaceutically acceptable salt thereof.

An embodiment of the present invention includes compounds of the formula Ie: 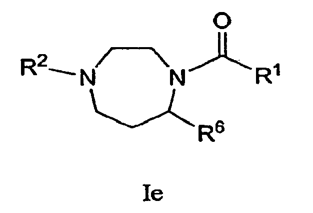 wherein R¹, R² and R⁶ are defined herein; or a pharmaceutically acceptable salt thereof.

An embodiment of the present invention includes compounds of the formula If: 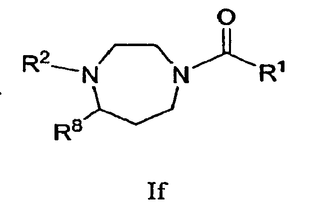 wherein R¹, R² and R⁸ are defined herein; or a pharmaceutically acceptable salt thereof.

An embodiment of the present invention includes compounds wherein X is -S02-.

An embodiment of the present invention includes compounds wherein X is -(C=O)-.

An embodiment of the present invention includes compounds wherein X is -(C=S)-.

An embodiment of the present invention includes compounds wherein R¹ is phenyl, which is unsubstituted or substituted with one or more of:
(1) halogen,
(2) hydroxyl,
(3) -Oₙ-C₁₋₆alkyl, where n is 0 or 1 (wherein ifn is 0, a bond is present) and where the alkyl is unsubstituted or substituted with one or more substituents selected from R¹³,
(4) -On-phenyl, where the phenyl is unsubstituted or substituted with one or more substituents selected from R¹³,
(5) -heterocycle, where the heterocycle is unsubstituted or substituted with one or more substituents selected from R¹³,
(6) -NR¹⁰R¹¹, wherein R¹⁰ and R¹¹ are independently selected from the group consisting of:
   (a) hydrogen, and
   (b) C₁₋₆alkyl, which is unsubstituted or substituted with one or more substituents selected from R¹³,
(7) -S(O)₂-NR¹⁰R¹¹,
(8) -CO₂H,
(9) -CN,
(10) -NO₂, an
(11) -B(OH)₂.

An embodiment of the present invention includes compounds wherein R¹ is phenyl, which is unsubstituted or substituted with one or more methyl, -CF₃, halo, -OCF₃, -OCH₃, -OCH₂CH₃, -CO₂CH₃ -CN, -N(CH₃), -NH(CH₂CH₃), -NO₂, -B(OH)₂, triazolyl or phenyl.

An embodiment of the present invention includes compounds wherein R¹ is phenyl, which is unsubstituted or substituted with one or more methyl, -CF₃, chloro, fluro, -OCF3, -OCH₃, -OCH₂CH₃, -CO₂CH₃, -B(OH)₂, triazolyl or phenyl.

An embodiment of the present invention includes compounds wherein R¹ is phenyl, which is unsubstituted or substituted with one or more methyl, -CF₃, fluro, -OCF₃, -OCH3, -CO₂CH₃, -B(OH)₂ or phenyl.

An embodiment of the present invention includes compounds wherein
R¹ is selected from the group consisting of:
(1) phenyl,
(2) biphenyl,
(3) 2,6-dimethoxyphenyl,
(4) 2,4-dichlorophenyl,
(5) 2,6-dichlorophenyl,
(6) 2,3-difluophenyl,
(7) 2,4-difluophenyl,
(8) 2,6-difluophenyl,
(9) 2-methoxy-4-methyl-phenyl,
(10) 3-methoxy-biphenyl,
(11) 3-methyl-biphenyl, and
(12) 5-methyl-2-triazolyl-phenyl.

An embodiment of the present invention includes compounds wherein R¹ is phenyl.

An embodiment of the present invention includes compounds wherein R¹ is 2,6-dimethoxyphenyl.

An embodiment of the present invention includes compounds wherein R² is heteroaryl, which is unsubstituted or substituted with one or more of:
(1) halogen,
(2) hydroxyl,
(3) -Oₙ-C₁₋₆alkyl, where n is 0 or 1 (wherein if n is 0, a bond is present) and where the alkyl is unsubstituted or substituted with one or more substituents selected from R¹³,
(4) -On-phenyl, where the phenyl is unsubstituted or substituted with one or more substituents selected from R¹³,
(5) -heterocycle, where the heterocycle is unsubstituted or substituted with one or more substituents selected from R¹³,
(6) -NR¹⁰R¹¹, wherein R¹⁰ and R¹¹ are independently selected from the group consisting of:
   (a) hydrogen,
   (b) C₁₋₆alkyl, which is unsubstituted or substituted with one or more substituents selected from R¹³,
(7) -S(O)₂-NR¹⁰R¹¹,
(8) -CO₂H,
(9) -CN, and
(10) -NO₂.

An embodiment of the present invention includes compounds wherein R2 is heteroaryl, which is unsubstituted or substituted with halogen, hydroxyl, C₁₋₆alkyl, -O-C₁₋₆alkyl or phenyl.

An embodiment of the present invention includes compounds wherein
R2 is selected from the group consisting of:
(1) benzimidazolyl,
(2) benzothiazolyl,
(3) benzoxazolyl,
(4) quinazolinyl,
(5) quinolinyl, and
(6) thiadiazolyl,
which is unsubstituted or substituted with halogen, hydroxyl, C₁₋₆alkyl, -O-C₁₋₆alkyl or phenyl.

An embodiment of the present invention includes compounds wherein R² is selected from the group consisting of:
(1) benzimidazol-2-yl,
(2) 1,3-benzothiazol-2-yl,
(3) 1,3-benzoxazol-2-yl,
(4) 2-quinazolinyl,
(5) 1-quinolin-2-yl, and
(6) 1,2,4-thiadiazol-5-yl,
which is unsubstituted or substituted with methyl, fluoro, chloro or phenyl.

An embodiment of the present invention includes compounds wherein R2 is benzothiazolyl, which is unsubstituted or substituted with chloro.

An embodiment of the present invention includes compounds wherein R2 is 6-chloro-benzothiazolyl.

An embodiment of the present invention includes compounds wherein R2 is benzoxazolyl.

An embodiment of the present invention includes compounds wherein R2 is quinazolinyl or quinolinyl.

An embodiment of the present invention includes compounds wherein R³, R⁴, R5, R6, R⁷ and R⁸ are independently selected from the group consisting of:
(1) hydrogen,
(2) -C₁₋₆alkyl, where the alkyl is unsubstituted or substituted with one or more substituents selected from R¹³,
or R⁴ and R⁵, or R⁶ and R⁷ are taken together to form a C₃₋₆cycloalkyl, where the cycloalkyl is unsubstituted or substituted with one or more substituents selected from R¹⁴,
with the proviso that at least one of R³, R⁴, R⁵, R⁶, R⁷ or R⁸ is other than hydrogen.

An embodiment of the present invention includes compounds wherein R³, R⁴, R⁵, R⁶, R⁷ and R⁸ are independently selected from the group consisting of:
(1) hydrogen, and
(2) -C₁₋₆alkyl,
or R⁴ and R⁵, or R⁶ and R⁷ are taken together to form a C₃₋₆cycloalkyl,
with the proviso that at least one of R³, R⁴, R⁵, R⁶, R⁷ or R⁸ is other than hydrogen.

An embodiment of the present invention includes compounds wherein R³ is -C₁₋₆alkyl, R⁴ is hydrogen, R⁵ is hydrogen, R⁶ is hydrogen, R⁷ is hydrogen, and R⁸ is hydrogen. Within this embodiment the present invention includes compounds wherein R³ is methyl, R⁴ is hydrogen, R⁵ is hydrogen, R⁶ is hydrogen, R⁷ is hydrogen, and R⁸ is hydrogen.

An embodiment of the present invention includes compounds wherein R³ is hydrogen, R⁴ is -C₁₋₆alkyl, R⁵ is hydrogen, R⁶ is hydrogen, R⁷ is hydrogen, and R⁸ is hydrogen. Within this embodiment the present invention includes compounds wherein R³ is hydrogen, R⁴ is methyl, R⁵ is hydrogen, R⁶ is hydrogen, R⁷ is hydrogen, and R⁸ is hydrogen.

An embodiment of the present invention includes compounds wherein R³ is hydrogen, R⁴ is hydrogen, R⁵ is hydrogen, R⁶ is -C₁₋₆alkyl, R⁷ is hydrogen, and R⁸ is hydrogen. Within this embodiment the present invention includes compounds wherein R³ is hydrogen, R⁴ is hydrogen, R⁵ is hydrogen, R⁶ is methyl, R⁷ is hydrogen, and R⁸ is hydrogen.

An embodiment of the present invention includes compounds wherein R³ is hydrogen, R⁴ is hydrogen, R⁵ is hydrogen, R⁶ is hydrogen, R⁷ is hydrogen, and R⁸ is -C₁₋₆alkyl. Within this embodiment the present invention includes compounds wherein R³ is hydrogen, R⁴ is hydrogen, R⁵ is hydrogen, R⁶ is hydrogen, R⁷ is hydrogen, and R⁸ is methyl.

An embodiment of the present invention includes compounds wherein R³ is hydrogen, R⁶ is hydrogen, R⁷ is hydrogen, R⁸ is hydrogen, and R⁴ and R⁵ are taken together to form a C₃₋₆cycloalkyl. Within this embodiment the present invention includes compounds wherein R³ is hydrogen, R⁶ is hydrogen, R⁷ is hydrogen, R⁸ is hydrogen, and R⁴ and R⁵ are taken together to form a cyclopropyl.

An embodiment of the present invention includes compounds wherein R³ is hydrogen, R⁴ is hydrogen, R⁵ is hydrogen, R⁸ is hydrogen, and R⁶ and R⁷ are taken together to form a C₃₋₆cycloalkyl. Within this embodiment the present invention includes compounds wherein R³ is hydrogen, R⁴ is hydrogen, R⁵ is hydrogen, R⁸ is hydrogen, and R⁶ and R⁷ are taken together to form a cyclopropyl.

Specific embodiments of the present invention include a compound which is selected from the group consisting of the subject compounds of the Examples herein or a pharmaceutically acceptable salt thereof.

The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography. The coupling reaction is often the formation of salts using an enantiomerically pure acid or base. The diasteromeric derivatives may then be converted to the pure enantiomers by cleavage of the added chiral residue. The racemic mixture of the compounds can also be separated directly by chromatographic methods utilizing chiral stationary phases, which methods are well known in the art. Alternatively, any enantiomer of a compound may be obtained by stereoselective synthesis using optically pure starting materials or reagents of known configuration by methods well known in the art.

As appreciated by those of skill in the art, halogen or halo as used herein are intended to include fluoro, chloro, bromo and iodo. Similarly, C₁₋₆, as in C₁₋₆alkyl is defined to identify the group as having 1, 2, 3, 4, 5 or 6 carbons in a linear or branched arrangement, such that C₁₋₈-alkyl specifically includes methyl, ethyl, n-propyl, iso-propyl, n-butyl, iso-butyl, tert-butyl, pentyl, and hexyl. A group which is designated as being independently substituted with substituents may be independently substituted with multiple numbers of such substituents. The term "heterocycle" as used herein includes both unsaturated and saturated heterocyclic moieties, wherein the unsaturated heterocyclic moieties (i.e. "heteroaryl") include benzoimidazolyl, benzimidazolonyl, benzofuranyl, benzofurazanyl, benzopyrazolyl, benzothiazolyl, benzotriazolyl, benzothiophenyl, benzoxazepin, benzoxazolyl, carbazolyl, carbolinyl, cinnolinyl, furanyl, imidazolyl, indolinyl, indolyl, indolazinyl, indazolyl, isobenzofuranyl, isoindolyl, isoquinolyl, isothiazolyl, isoxazolyl, naphthpyridinyl, oxadiazolyl, oxazolyl, oxazoline, isoxazoline, oxetanyl, pyrazinyl, pyrazolyl, pyridazinyl, pyridopyridinyl, pyridazinyl, pyridyl, pyrimidyl, pyrrolyl, quinazolinyl, quinolyl, quinoxalinyl, tetrazolyl, tetrazolopyridyl, thiadiazolyl, thiazolyl, thienyl, triazolyl, and N-oxides thereof, and wherein the saturated heterocyclic moieties include azetidinyl, 1,4-dioxanyl, hexahydroazepinyl, piperazinyl, piperidinyl, pyridin-2-onyl, pyrrolidinyl, morpholinyl, tetrahydrofuranyl, thiomorpholinyl, and tetrahydrothienyl, and N-oxides thereof.

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts in the solid form may exist in more than one crystal structure, and may also be in the form of hydrates. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylene-diamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, parnoic, pantothenic, phosphoric, succinic, sulfuric, tartaric, p-toluenesulfonic acid, and the like. Particularly preferred are citric, hydrobromic, hydrochloric, maleic, phosphoric, sulfuric, fumaric, and tartaric acids. It will be understood that, as used herein, references to the compounds of Formula I are meant to also include the pharmaceutically acceptable salts.

Exemplifying the invention is the use of the compounds disclosed in the Examples and herein. Specific compounds within the present invention include a compound which selected from the group consisting of the compounds disclosed in the following Examples and pharmaceutically acceptable salts thereof and individual diastereomers thereof.

The subject compounds are useful in a method of antagonizing orexin receptor activity in a patient such as a mammal in need of such inhibition comprising the administration of an effective amount of the compound. The present invention is directed to the compounds disclosed herein for use as antagonists of orexin receptor activity. In addition to primates, especially humans, a variety of other mammals can be treated according to the method of the present invention. The present invention is directed to a compound of the present invention or a pharmaceutically acceptable salt thereof for use in medicine. The present invention is further directed to a use of a compound of the present invention or a pharmaceutically acceptable salt thereof for the manufacture of a medicament for antagonizing orexin receptor activity or treating the disorders and diseases noted herein in humans and animals.

The subject treated in the present methods is generally a mammal, preferably a human being, male or female. The term "therapeutically effective amount" means the amount of the subject compound that will elicit the biological or medical response of a tissue, system, animal or human that is being sought by the researcher, veterinarian, medical doctor or other clinician. It is recognized that one skilled in the art may affect the neurological and psychiatric disorders by treating a patient presently afflicted with the disorders or by prophylactically treating a patient afflicted with the disorders with an effective amount of the compound of the present invention. As used herein, the terms "treatment" and "treating" refer to all processes wherein there may be a slowing, interrupting, arresting, controlling, or stopping of the progression of the neurological and psychiatric disorders described herein, but does not necessarily indicate a total elimination of all disorder symptoms, as well as the prophylactic therapy of the mentioned conditions, particularly in a patient who is predisposed to such disease or disorder. The terms "administration of" and or "administering a" compound should be understood to mean providing a compound of the invention or a prodrug of a compound of the invention to the individual in need thereof.

The term "composition" as used herein is intended to encompass a product comprising the specified ingredients in the specified amounts, as well as any product which results, directly or indirectly, from combination of the specified ingredients in the specified amounts. Such term in relation to pharmaceutical composition, is intended to encompass a product comprising the active ingredient(s), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier. By "pharmaceutically acceptable" it is meant the carrier, diluent or excipient must be compatible with the other ingredients of the formulation and not deleterious to the recipient thereof.

The utility of the compounds in accordance with the present invention as orexin receptor OX1R and/or OX2R antagonists may be readily determined without undue experimentation by methodology well known in the art, including the "FLIPR Ca²⁺ Flux Assay" (Okumura et al., Biochem. Biophys. Res. Comm. 280:976-981,2001). In a typical experiment the OX1 and OX2 receptor antagonistic activity of the compounds of the present invention was determined in accordance with the following experimental method. For intracellular calcium measurements, Chinese hamster ovary (CHO) cells expressing the rat orexin-1 receptor or the human orexin-2 receptor, are grown in Iscove's modified DMEM containing 2 mM L-glutamine, 0.5 g/ml G418, 1% hypoxanthine-thymidine supplement, 100 U/ml penicillin, 100 ug/ml streptomycin and 10 % heat-inactivated fetal calf serum (FCS). The cells are seeded at 20,000 cells / well into Becton-Dickinson black 384-well clear bottom sterile plates coated with poly-D-lysine. All reagents were from GIBCO-Invitrogen Corp. The seeded plates are incubated overnight at 37°C and 5% CO2. Ala^{6,12} human orexin-A as the agonist is prepared as a 1 mM stock solution in 1% bovine serum albumin (BSA) and diluted in assay buffer (HBSS containing 20 mM HEPES, 0.1% BSA and 2.5mM probenecid, pH7.4) for use in the assay at a final concentration of 70pM. Test compounds are prepared as 10 mM stock solution in DMSO, then diluted in 384-well plates, first in DMSO, then assay buffer. On the day of the assay, cells are washed 3 times with 100 ul assay buffer and then incubated for 60 min (37° C, 5% CO2) in 60 ul assay buffer containing 1 uM Fluo-4AM ester, 0.02 % pluronic acid, and 1 % BSA. The dye loading solution is then aspirated and cells are washed 3 times with 100 ul assay buffer. 30 ul of that same buffer is left in each well. Within the Fluorescent Imaging Plate Reader (FLIPR, Molecular Devices), test compounds are added to the plate in a volume of 25 ul , incubated for 5 min and finally 25 ul of agonist is added. Fluorescence is measured for each well at 1 second intervals for 5 minutes and the height of each fluorescence peak is compared to the height of the fluorescence peak induced by 70 pM Ala^{6,12} orexin-A with buffer in place of antagonist. For each antagonist, IC50 value (the concentration of compound needed to inhibit 50 % of the agonist response) is determined. The intrinsic orexin receptor antagonist activity of a compound which may be used in the present invention may be determined by these assays.

In particular, the compounds of the following examples had activity in antagonizing the rat orexin-1 receptor and/or the human orexin-2 receptor in the aforementioned assays, generally with an IC₅₀ of less than about 50 µM. Preferred compounds within the present invention had activity in antagonizing the rat orexin-1 receptor and/or the human orexin-2 receptor in the aforementioned assays with an IC₅₀ of less than about 100 nM. Such a result is indicative of the intrinsic activity of the compounds in use as antagonists of orexin-1 receptor and/or the orexin-2 receptor. The present invention also includes compounds within the generic scope of the invention which possess activity as agonists of the orexin-1 receptor and/or the orexin-2 receptor.

The orexin receptors have been implicated in a wide range of biological functions. This has suggested a potential role for these receptors in a variety of disease processes in humans or other species. The compounds of the present invention have utility in treating, preventing, ameliorating, controlling or reducing the risk of a variety of neurological and psychiatric disorders associated with orexin receptors, including one or more of the following conditions or diseases: sleep disorders, sleep disturbances, including enhancing sleep quality, improving sleep quality, increasing sleep efficiency, augmenting sleep maintenance; increasing the value which is calculated from the time that a subject sleeps divided by the time that a subject is attempting to sleep; improving sleep initiation; decreasing sleep latency or onset (the time it takes to fall asleep); decreasing difficulties in falling asleep; increasing sleep continuity; decreasing the number of awakenings during sleep; decreasing intermittent wakings during sleep; decreasing nocturnal arousals; decreasing the time spent awake following the initial onset of sleep; increasing the total amount of sleep; reducing the fragmentation of sleep; altering the timing, frequency or duration of REM sleep bouts; altering the timing, frequency or duration of slow wave (i.e. stages 3 or 4) sleep bouts; increasing the amount and percentage of stage 2 sleep; promoting slow wave sleep; enhancing EEG-delta activity during sleep; decreasing nocturnal arousals, especially early morning awakenings; increasing daytime alertness; reducing daytime drowsiness; treating or reducing excessive daytime sleepiness; increasing satisfaction with the intensity of sleep; increasing sleep maintenance; idiopathic insomnia; sleep problems; insomnia, hypersomnia, idiopathic hypersomnia, repeatability hypersomnia, intrinsic hypersomnia, narcolepsy, interrupted sleep, sleep apnea, wakefulness, nocturnal myoclonus, REM sleep interruptions, jet-lag, shift workers' sleep disturbances, dyssomnias, night terror, insomnias associated with depression, emotional/mood disorders, Alzheimer's disease or cognitive impairment, as well as sleep walking and enuresis, and sleep disorders which accompany aging; Alzheimer's sundowning; conditions associated with circadian rhythmicity as well as mental and physical disorders associated with travel across time zones and with rotating shift-work schedules, conditions due to drugs which cause reductions in REM sleep as a side effect; fibromyalgia; syndromes which are manifested by non-restorative sleep and muscle pain or sleep apnea which is associated with respiratory disturbances during sleep; conditions which result from a diminished quality of sleep; eating disorders associated with excessive food intake and complications associated therewith, compulsive eating disorders, obesity (due to any cause, whether genetic or environmental), obesity-related disorders including overeating and bulimia nervosa, hypertension, diabetes, elevated plasma insulin concentrations and insulin resistance, dyslipidemias, hyperlipidemia, endometrial, breast, prostate and colon cancer, osteoarthritis, obstructive sleep apnea, cholelithiasis, gallstones, heart disease, abnormal heart rhythms and arrythmias, myocardial infarction, congestive heart failure, coronary heart disease, sudden death, stroke, polycystic ovary disease, craniopharyngioma, the Prader-Willi Syndrome, Frohlich's syndrome, GH-deficient subjects, normal variant short stature, Turner's syndrome, and other pathological conditions showing reduced metabolic activity or a decrease in resting energy expenditure as a percentage of total fat-free mass, e.g, children with acute lymphoblastic leukemia, metabolic syndrome, also known as syndrome X, insulin resistance syndrome, reproductive hormone abnormalities, sexual and reproductive dysfunction, such as impaired fertility, infertility, hypogonadism in males and hirsutism in females, fetal defects associated with maternal obesity, gastrointestinal motility disorders, such as obesity-related gastro-esophageal reflux, respiratory disorders, such as obesity-hypoventilation syndrome (Pickwickian syndrome), breathlessness, cardiovascular disorders, inflammation, such as systemic inflammation of the vasculature, arteriosclerosis, hypercholesterolemia, hyperuricaemia, lower back pain, gallbladder disease, gout, kidney cancer, increased anesthetic risk, reducing the risk of secondary outcomes of obesity, such as reducing the risk of left ventricular hypertrophy; diseases or disorders where abnormal oscillatory activity occurs in the brain, including depression, migraine, neuropathic pain, Parkinson's disease, psychosis and schizophrenia, as well as diseases or disorders where there is abnormal coupling of activity, particularly through the thalamus; enhancing cognitive function; enhancing memory; increasing memory retention; increasing immune response; increasing immune function; hot flashes; night sweats; extending life span; schizophrenia; muscle-related disorders that are controlled by the excitation/relaxation rhythms imposed by the neural system such as cardiac rhythm and other disorders of the cardiovascular system; conditions related to proliferation of cells such as vasodilation or vasorestriction and blood pressure; cancer; cardiac arrhythmia; hypertension; congestive heart failure; conditions of the genital/urinary system; disorders of sexual function and fertility; adequacy of renal function; responsivity to anesthetics; mood disorders, such as depression or more particularly depressive disorders, for example, single episodic or recurrent major depressive disorders and dysthymic disorders, or bipolar disorders, for example, bipolar I disorder, bipolar II disorder and cyclothymic disorder, mood disorders due to a general medical condition, and substance-induced mood disorders; anxiety disorders including acute stress disorder, agoraphobia, generalized anxiety disorder, obsessive-compulsive disorder, panic attack, panic disorder, post-traumatic stress disorder, separation anxiety disorder, social phobia, specific phobia, substance-induced anxiety disorder and anxiety due to a general medical condition; acute neurological and psychiatric disorders such as cerebral deficits subsequent to cardiac bypass surgery and grafting, stroke, ischemic stroke, cerebral ischemia, spinal cord trauma, head trauma, perinatal hypoxia, cardiac arrest, hypoglycemic neuronal damage; Huntington's Chorea; amyotrophic lateral sclerosis; multiple sclerosis; ocular damage; retinopathy; cognitive disorders; idiopathic and drug-induced Parkinson's disease; muscular spasms and disorders associated with muscular spasticity including tremors, epilepsy, convulsions; cognitive disorders including dementia (associated with Alzheimer's disease, ischemia, trauma, vascular problems or stroke, HIV disease, Parkinson's disease, Huntington's disease, Pick's disease, Creutzfeldt-Jacob disease, perinatal hypoxia, other general medical conditions or substance abuse); delirium, amnestic disorders or age related cognitive decline; schizophrenia or psychosis including schizophrenia (paranoid, disorganized, catatonic or undifferentiated), schizophreniform disorder, schizoaffective disorder, delusional disorder, brief psychotic disorder, shared psychotic disorder, psychotic disorder due to a general medical condition and substance-induced psychotic disorder; substance-related disorders and addictive behaviors (including substance-induced delirium, persisting dementia, persisting amnestic disorder, psychotic disorder or anxiety disorder; tolerance, dependence or withdrawal from substances including alcohol, amphetamines, cannabis, cocaine, hallucinogens, inhalants, nicotine, opioids, phencyclidine, sedatives, hypnotics or anxiolytics); movement disorders, including akinesias and akinetic-rigid syndromes (including Parkinson's disease, drug-induced parkinsonism, postencephalitic parkinsonism, progressive supranuclear palsy, multiple system atrophy, corticobasal degeneration, parkinsonism-ALS dementia complex and basal ganglia calcification), chronic fatigue syndrome, fatigue, including Parkinson's fatigue, multiple sclerosis fatigue, fatigue caused by a sleep disorder or a circadian rhythm disorder, medication-induced parkinsonism (such as neuroleptic-induced parkinsonism, neuroleptic malignant syndrome, neuroleptic-induced acute dystonia, neuroleptic-induced acute akathisia, neuroleptic-induced tardive dyskinesia and medication-induced postural tremor), Gilles de la Tourette's syndrome, epilepsy, and dyskinesias [including tremor (such as rest tremor, essential tremor, postural tremor and intention tremor), chorea (such as Sydenham's chorea, Huntington's disease, benign hereditary chorea, neuroacanthocytosis, symptomatic chorea, drug-induced chorea and hemiballism), myoclonus (including generalised myoclonus and focal myoclonus), tics (including simple tics, complex tics and symptomatic tics), restless leg syndrome and dystonia (including generalised dystonia such as iodiopathic dystonia, drug-induced dystonia, symptomatic dystonia and paroxymal dystonia, and focal dystonia such as blepharospasm, oromandibular dystonia, spasmodic dysphonia, spasmodic torticollis, axial dystonia, dystonic writer's cramp and hemiplegic dystonia); attention deficit/hyperactivity disorder (ADHD); conduct disorder; migraine (including migraine headache); urinary incontinence; substance tolerance, substance withdrawal (including, substances such as opiates, nicotine, tobacco products, alcohol, benzodiazepines, cocaine, sedatives, hypnotics, etc.); psychosis; schizophrenia; anxiety (including generalized anxiety disorder, panic disorder, and obsessive compulsive disorder); mood disorders (including depression, mania, bipolar disorders); trigeminal neuralgia; hearing loss; tinnitus; neuronal damage including ocular damage; retinopathy; macular degeneration of the eye; emesis; brain edema; pain, including acute and chronic pain states, severe pain, intractable pain, inflammatory pain, neuropathic pain, post-traumatic pain, bone and joint pain (osteoarthritis), repetitive motion pain, dental pain, cancer pain, myofascial pain (muscular injury, fibromyalgia), perioperative pain (general surgery, gynecological), chronic pain, neuropathic pain, post-traumatic pain, trigeminal neuralgia, migraine and migraine headache.

Thus, in preferred embodiments the present invention provides methods for: enhancing the quality of sleep; augmenting sleep maintenance; increasing REM sleep; increasing stage 2 sleep; decreasing fragmentation of sleep patterns; treating insomnia; enhancing cognition; increasing memory retention; treating or controlling obesity; treating or controlling depression; treating, controlling, ameliorating or reducing the risk of epilepsy, including absence epilepsy; treating or controlling pain, including neuropathic pain; treating or controlling Parkinson's disease; treating or controlling psychosis; or treating, controlling, ameliorating or reducing the risk of schizophrenia, in a mammalian patient in need thereof which comprises administering to the patient a therapeutically effective amount of a compound of the present invention.

The subject compounds are further useful in a method for the prevention, treatment, control, amelioration, or reducation of risk of the diseases, disorders and conditions noted herein. The dosage of active ingredient in the compositions of this invention may be varied, however, it is necessary that the amount of the active ingredient be such that a suitable dosage form is obtained. The active ingredient may be administered to patients (animals and human) in need of such treatment in dosages that will provide optimal pharmaceutical efficacy. The selected dosage depends upon the desired therapeutic effect, on the route of administration, and on the duration of the treatment. The dose will vary from patient to patient depending upon the nature and severity of disease, the patient's weight, special diets then being followed by a patient, concurrent medication, and other factors which those skilled in the art will recognize. Generally, dosage levels of between 0.0001 to 10 mg/kg. of body weight daily are administered to the patient, e.g., humans and elderly humans, to obtain effective antagonism of orexin receptors. The dosage range will generally be about 0.5 mg to 1.0 g. per patient per day which may be administered in single or multiple doses. Preferably, the dosage range will be about 0.5 mg to 500 mg per patient per day; more preferably about 0.5 mg to 200 mg per patient per day; and even more preferably about 5 mg to 50 mg per patient per day. Pharmaceutical compositions of the present invention may be provided in a solid dosage formulation preferably comprising about 0.5 mg to 500 mg active ingredient, more preferably comprising about 1 mg to 250 mg active ingredient. The pharmaceutical composition is preferably provided in a solid dosage formulation comprising about 1 mg, 5 mg, 10 mg, 25 mg, 50 mg, 100 mg, 200 mg or 250 mg active ingredient. For oral administration, the compositions are preferably provided in the form of tablets containing 1.0 to 1000 milligrams of the active ingredient, particularly 1, 5, 10, 15, 20, 25, 50, 75, 100, 150, 200, 250, 300, 400, 500, 600, 750, 800, 900, and 1000 milligrams of the active ingredient for the symptomatic adjustment of the dosage to the patient to be treated. The compounds may be administered on a regimen of 1 to 4 times per day, preferably once or twice per day.

The compounds of the present invention may be used in combination with one or more other drugs in the treatment, prevention, control, amelioration, or reduction of risk of diseases or conditions for which compounds of the present invention or the other drugs may have utility, where the combination of the drugs together are safer or more effective than either drug alone. Such other drug(s) may be administered, by a route and in an amount commonly used therefor, contemporaneously or sequentially with a compound of the present invention. When a compound of the present invention is used contemporaneously with one or more other drugs, a pharmaceutical composition in unit dosage form containing such other drugs and the compound of the present invention is preferred. However, the combination therapy may also includes therapies in which the compound of the present invention and one or more other drugs are administered on different overlapping schedules. It is also contemplated that when used in combination with one or more other active ingredients, the compounds of the present invention and the other active ingredients may be used in lower doses than when each is used singly. Accordingly, the pharmaceutical compositions of the present invention include those that contain one or more other active ingredients, in addition to a compound of the present invention. The above combinations include combinations of a compound of the present invention not only with one other active compound, but also with two or more other active compounds.

Likewise, compounds of the present invention may be used in combination with other drugs that are used in the prevention, treatment, control, amelioration, or reduction of risk of the diseases or conditions for which compounds of the present invention are useful. Such other drugs may be administered, by a route and in an amount commonly used therefor, contemporaneously or sequentially with a compound of the present invention. When a compound of the present invention is used contemporaneously with one or more other drugs, a pharmaceutical composition containing such other drugs in addition to the compound of the present invention is preferred. Accordingly, the pharmaceutical compositions of the present invention include those that also contain one or more other active ingredients, in addition to a compound of the present invention.

The weight ratio of the compound of the compound of the present invention to the second active ingredient may be varied and will depend upon the effective dose of each ingredient. Generally, an effective dose of each will be used. Thus, for example, when a compound of the present invention is combined with another agent, the weight ratio of the compound of the present invention to the other agent will generally range from about 1000:1 to about 1:1000, preferably about 200:1 to about 1:200. Combinations of a compound of the present invention and other active ingredients will generally also be within the aforementioned range, but in each case, an effective dose of each active ingredient should be used. In such combinations the compound of the present invention and other active agents may be administered separately or in conjunction. In addition, the administration of one element may be prior to, concurrent to, or subsequent to the administration of other agent(s).

The compounds of the present invention may be administered in conbination with other compounds which are known in the art to be useful for enhancing sleep quality and preventing and treating sleep disorders and sleep disturbances, including e.g., sedatives, hypnotics, anxiolytics, antipsychotics, antianxiety agents, antihistamines, benzodiazepines, barbiturates, cyclopyrrolones, GABA agonists, 5HT-2 antagonists including 5HT-2A antagonists and 5HT-2A/2C antagonists, histamine antagonists including histamine H3 antagonists, histamine H3 inverse agonists, imidazopyridines, minor tranquilizers, melatonin agonists and antagonists, melatonergic agents, other orexin antagonists, orexin agonists, prokineticin agonists and antagonists, pyrazolopyrimidines, T-type calcium channel antagonists, triazolopyridines, and the like, such as: adinazolam, allobarbital, alonimid, alprazolam, amitriptyline, amobarbital, amoxapine, armodafinil, APD-125, bentazepam, benzoctamine, brotizolam, bupropion, busprione, butabarbital, butalbital, capromorelin, capuride, carbocloral, chloral betaine, chloral hydrate, chlordiazepoxide, clomipramine, clonazepam, cloperidone, clorazepate, clorethate, clozapine, conazepam, cyprazepam, desipramine, dexclamol, diazepam, dichloralphenazone, divalproex, diphenhydramine, doxepin, EMD-281014, eplivanserin, estazolam, eszopiclone, ethchlorynol, etomidate, fenobam, flunitrazepam, flurazepam, fluvoxamine, fluoxetine, fosazepam, gaboxadol, glutethimide, halazepam, hydroxyzine, ibutamoren, imipramine, indiplon, lithium, lorazepam, lormetazepam, LY-156735, maprotiline, MDL-100907, mecloqualone, melatonin, mephobarbital, meprobamate, methaqualone, methyprylon, midaflur, midazolam, modafinil, nefazodone, NGD-2-73, nisobamate, nitrazepam, nortriptyline, oxazepam, paraldehyde, paroxetine, pentobarbital, perlapine, perphenazine, phenelzine, phenobarbital, prazepam, promethazine, propofol, protriptyline, quazepam, ramelteon, reclazepam, roletamide, secobarbital, sertraline, suproclone, TAK-375, temazepam, thioridazine, tiagabine, tracazolate, tranylcypromaine, trazodone, triazolam, trepipam, tricetamide, triclofos, trifluoperazine, trimetozine, trimipramine, uldazepam, venlafaxine, zaleplon, zolazepam, zopiclone, zolpidem, and salts thereof, and combinations thereof, and the like, or the compound of the present invention may be administered in conjunction with the use of physical methods such as with light therapy or electrical stimulation.

In another embodiment, the subject compound may be employed in combination with other compounds which are known in the art, either administered separately or in the same pharmaceutical compositions, include, but are not limited to: insulin sensitizers including (i) PPARγ antagonists such as glitazones (e.g. ciglitazone; darglitazone; englitazone; isaglitazone (MCC-555); pioglitazone; rosiglitazone; troglitazone; tularik; BRL49653; CLX-0921; 5-BTZD), GW-0207, LG-100641, and LY-300512, and the like); (iii) biguanides such as metformin and phenformin; (b) insulin or insulin mimetics, such as biota, LP-100, novarapid, insulin detemir, insulin lispro, insulin glargine, insulin zinc suspension (lente and ultralente); Lys-Pro insulin, GLP-1 (73-7) (insulintropin); and GLP-1 (7-36)-NH₂); (c) sulfonylureas, such as acetohexamide; chlorpropamide; diabinese; glibenclamide; glipizide; glyburide; glimepiride; gliclazide; glipentide; gliquidone; glisolamide; tolazamide; and tolbutamide; (d) α-glucosidase inhibitors, such as acarbose, adiposine; camiglibose; emiglitate; miglitol; voglibose; pradimicin-Q; salbostatin; CKD-711; MDL-25,637; MDL-73,945; and MOR 14, and the like; (e) cholesterol lowering agents such as (i) HMG-CoA reductase inhibitors (atorvastatin, itavastatin, fluvastatin, lovastatin, pravastatin, rivastatin, rosuvastatin, simvastatin, and other statins), (ii) bile acid absorbers/sequestrants, such as cholestyramine, colestipol, dialkylaminoalkyl derivatives of a cross-linked dextran; Colestid®; LoCholest®, and the like, (ii) nicotinyl alcohol, nicotinic acid or a salt thereof, (iii) proliferator-activater receptor α agonists such as fenofibric acid derivatives (gemfibrozil, clofibrate, fenofibrate and benzafibrate), (iv) inhibitors of cholesterol absorption such as stanol esters, beta-sitosterol, sterol glycosides such as tiqueside; and azetidinones such as ezetimibe, and the like, and (acyl CoA:cholesterol acyltransferase (ACAT)) inhibitors such as avasimibe, and melinamide, (v) anti-oxidants, such as probucol, (vi) vitamin E, and (vii) thyromimetics; (f) PPARα agonists such as beclofibrate, benzafibrate, ciprofibrate, clofibrate, etofibrate, fenofibrate, and gemfibrozil; and other fibric acid derivatives, such as Atromid®, Lopid® and Tricor®, and the like, and PPARα agonists as described in WO 97/36579 by Glaxo; (g) PPARδ agonists; (h) PPAR α/δ agonists, such as muraglitazar, and the compounds disclosed in US 6,414,002; and (i) anti-obesity agents, such as (1) growth hormone secretagogues, growth hormone secretagogue receptor agonists/antagonists, such as NN703, hexarelin, MK-0677, SM-130686, CP-424,391, L-692,429, and L-163,255; (2) protein tyrosine phosphatase-1B (PTP-1B) inhibitors; (3) cannabinoid receptor ligands, such as cannabinoid CB₁ receptor antagonists or inverse agonists, such as rimonabant (Sanofi Synthelabo), AMT-251, and SR-14778 and SR 141716A (Sanofi Synthelabo), SLV-319 (Solvay), BAY 65-2520 (Bayer); (4) anti-obesity serotonergic agents, such as fenfluramine, dexfenfluramine, phentermine, and sibutramine; (5) β3-adrenoreceptor agonists, such as AD9677/TAK677 (Dainippon/Takeda), CL-316,243, SB 418790, BRL-37344, L-796568, BMS-196085, BRL-35135A, CGP12177A, BTA-243, Trecadrine, Zeneca D7114, SR 59119A; (6) pancreatic lipase inhibitors, such as orlistat (Xenical®), Triton WR1339, RHC80267, lipstatin, tetrahydrolipstatin, teasaponin, diethylumbelliferyl phosphate; (7) neuropeptide Y1 antagonists, such as BIBP3226, J-115814, BIBO 3304, LY-357897, CP-671906, GI-264879A; (8) neuropeptide Y5 antagonists, such as GW-569180A, GW-594884A, GW-587081X, GW-548118X, FR226928, FR 240662, FR252384, 1229U91, GI-264879A, CGP71683A, LY-377897, PD-160170, SR-120562A, SR-120819A and JCF-104; (9) melanin-concentrating hormone (MCH) receptor antagonists; (10) melanin-concentrating hormone 1 receptor (MCH1R) antagonists, such as T-226296 (Takeda); (11) melanin-concentrating hormone 2 receptor (MCH2R) agonist/antagonists; (12) orexin receptor antagonists, such as SB-334867-A, and those disclosed in patent publications herein; (13) serotonin reuptake inhibitors such as fluoxetine, paroxetine, and sertraline; (14) melanocortin agonists, such as Melanotan II; (15) other Mc4r (melanocortin 4 receptor) agonists, such as CHIR86036 (Chiron), ME-10142, and ME-10145 (Melacure), CHIR86036 (Chiron); PT-141, and PT-14 (Palatin); (16) 5HT-2 agonists; (17) 5HT2C (serotonin receptor 2C) agonists, such as BVT933, DPCA37215, WAY161503, R-1065; (18) galanin antagonists; (19) CCK agonists; (20) CCK-A (cholecystokinin-A) agonists, such as AR-R 15849, GI 181771, JMV-180, A-71378, A-71623 and SR14613; (22) corticotropin-releasing hormone agonists; (23) histamine receptor-3 (H3) modulators; (24) histamine receptor-3 (H3) antagonists/inverse agonists, such as hioperamide, 3-(1H-imidazol-4-yl)propyl N-(4-pentenyl)carbamate, clobenpropit, iodophenpropit, imoproxifan, GT2394 (Gliatech), and O-[3-(1H-imidazol-4-yl)propanol]-carbamates; (25) β-hydroxy steroid dehydrogenase-1 inhibitors (β-HSD-1); 26) PDE (phosphodiesterase) inhibitors, such as theophylline, pentoxifylline, zaprinast, sildenafil, amrinone, milrinone, cilostamide, rolipram, and cilomilast; (27) phosphodiesterase-3B (PDE3B) inhibitors; (28) NE (norepinephrine) transport inhibitors, such as GW 320659, despiramine, talsupram, and nomifensine; (29) ghrelin receptor antagonists; (30) leptin, including recombinant human leptin (PEG-OB, Hoffman La Roche) and recombinant methionyl human leptin (Amgen); (31) leptin derivatives; (32) BRS3 (bombesin receptor subtype 3) agonists such as [D-Phe6,beta-Ala11 ,Phe13,Nle14]Bn(6-14) and [D-Phe6,Phe13]Bn(6-13)propylamide, and those compounds disclosed in Pept. Sci. 2002 Aug; 8(8): 461-75); (33) CNTF (Ciliary neurotrophic factors), such as GI-181771 (Glaxo-SmithKline), SR146131 (Sanofi Synthelabo), butabindide, PD170,292, and PD 149164 (Pfizer); (34) CNTF derivatives, such as axokine (Regeneron); (35) monoamine reuptake inhibitors, such as sibutramine; (36) UCP-1 (uncoupling protein-1), 2, or 3 activators, such as phytanic acid, 4-[(E)-2-(5,6,7,8-tetrahydro-5,5,8,8-tetramethyl-2-napthalenyl)-1-propenyl]benzoic acid (TTNPB), retinoic acid; (37) thyroid hormone β agonists, such as KB-2611 (KaroBioBMS); (38) FAS (fatty acid synthase) inhibitors, such as Cerulenin and C75; (39) DGAT1 (diacylglycerol acyltransferase 1) inhibitors; (40) DGAT2 (diacylglycerol acyltransferase 2) inhibitors; (41) ACC2 (acetyl-CoA carboxylase-2) inhibitors; (42) glucocorticoid antagonists; (43) acyl-estrogens, such as oleoyl-estrone, disclosed in del Mar-Grasa, M. et al., Obesity Research, 9:202-9 (2001); (44) dipeptidyl peptidase IV (DP-IV) inhibitors, such as isoleucine thiazolidide, valine pyrrolidide, NVP-DPP728, LAF237, MK-431, P93/01, TSL 225, TMC-2A/2B/2C, FE 999011, P9310/K364, VIP 0177, SDZ 274-444; (46) dicarboxylate transporter inhibitors; (47) glucose transporter inhibitors; (48) phosphate transporter inhibitors; (49) Metformin (Glucophage®); and (50) Topiramate (Topimax®); and (50) peptide YY, PYY 3-36, peptide YY analogs, derivatives, and fragments such as BIM-43073D, BIM-43004C (Olitvak, D.A. et al., Dig. Dis. Sci. 44(3):643-48 (1999)); (51) Neuropeptide Y2 (NPY2) receptor agonists such NPY3-36, N acetyl [Leu(28,31)] NPY 24-36, TASP-V, and cyclo-(28/32)-Ac-[Lys28-Glu32]-(25-36)-pNPY; (52) Neuropeptide Y4 (NPY4) agonists such as pancreatic peptide (PP), and other Y4 agonists such as 1229U91; (54) cyclooxygenase-2 inhibitors such as etoricoxib, celecoxib, valdecoxib, parecoxib, lumiracoxib, BMS347070, tiracoxib or JTE522, ABT963, CS502 and GW406381, and pharmaceutically acceptable salts thereof; (55) Neuropeptide Y1 (NPY1) antagonists such as BIBP3226, J-115814, BIBO 3304, LY-357897, CP-671906, GI-264879A; (56) Opioid antagonists such as nalmefene (Revex ®), 3-methoxynaltrexone, naloxone, naltrexone; (57) 11β HSD-1(11-beta hydroxy steroid dehydrogenase type 1) inhibitor such as BVT 3498, BVT 2733; (58) aminorex; (59) amphechloral; (60) amphetamine; (61) benzphetamine; (62) chlorphentermine; (63) clobenzorex; (64) cloforex; (65) clominorex; (66) clortermine; (67) cyclexedrine; (68) dextroamphetamine; (69) diphemethoxidine, (70) N-ethylamphetamine; (71) fenbutrazate; (72) fenisorex; (73) fenproporex; (74) fludorex; (75) fluminorex; (76) furfurylmethylamphetamine; (77) levamfetamine; (78) levophacetoperane; (79) mefenorex; (80) metamfepramone; (81) methamphetamine; (82) norpseudoephedrine; (83) pentorex; (84) phendimetrazine; (85) phenmetrazine; (86) picilorex; (87) phytopharm 57; and (88) zonisamide.

In another embodiment, the subject compound may be employed in combination with an anti-depressant or anti-anxiety agent, including norepinephrine reuptake inhibitors (including tertiary amine tricyclics and secondary amine tricyclics), selective serotonin reuptake inhibitors (SSRIs), monoamine oxidase inhibitors (MAOIs), reversible inhibitors of monoamine oxidase (RIMAs), serotonin and noradrenaline reuptake inhibitors (SNRIs), corticotropin releasing factor (CRF) antagonists, α-adrenoreceptor antagonists, neurokinin-1 receptor antagonists, atypical anti-depressants, benzodiazepines, 5-HT_{1A} agonists or antagonists, especially 5-HT_{1A} partial agonists, and corticotropin releasing factor (CRF) antagonists. Specific agents include: amitriptyline, clomipramine, doxepin, imipramine and trimipramine; amoxapine, desipramine, maprotiline, nortriptyline and protriptyline; fluoxetine, fluvoxamine, paroxetine and sertraline; isocarboxazid, phenelzine, tranylcypromine and selegiline; moclobemide: venlafaxine; aprepitant; bupropion, lithium, nefazodone, trazodone and viloxazine; alprazolam, chlordiazepoxide, clonazepam, chlorazepate, diazepam, halazepam, lorazepam, oxazepam and prazepam; buspirone, flesinoxan, gepirone and ipsapirone, and pharmaceutically acceptable salts thereof.

In another embodiment, the subject compound maybe employed in combination with anti-Alzheimer's agents; beta-secretase inhibitors; gamma-secretase inhibitors; growth hormone secretagogues; recombinant growth hormone; HMG-CoA reductase inhibitors; NSAID's including ibuprofen; vitamin E; anti-amyloid antibodies; CB-1 receptor antagonists or CB-1 receptor inverse agonists; antibiotics such as doxycycline and rifampin; N-methyl-D-aspartate (NMDA) receptor antagonists, such as memantine; cholinesterase inhibitors such as galantamine, rivastigmine, donepezil, and tacrine; growth hormone secretagogues such as ibutamoren, ibutamoren mesylate, and capromorelin; histamine H₃ antagonists; AMPA agonists; PDE IV inhibitors; GABA_{A} inverse agonists; or neuronal nicotinic agonists.

In another embodiment, the subject compound may be employed in combination with sedatives, hypnotics, anxiolytics, antipsychotics, antianxiety agents, cyclopyrrolones, imidazopyridines, pyrazolopyrimidines, minor tranquilizers, melatonin agonists and antagonists, melatonergic agents, benzodiazepines, barbiturates, 5HT-2 antagonists, and the like, such as: adinazolam, allobarbital, alonimid, alprazolam, amitriptyline, amobarbital, amoxapine, bentazepam, benzoctamine, brotizolam, bupropion, busprione, butabarbital, butalbital, capuride, carbocloral, chloral betaine, chloral hydrate, chlordiazepoxide, clomipramine, clonazepam, cloperidone, clorazepate, clorethate, clozapine, cyprazepam, desipramine, dexclamol, diazepam, dichloralphenazone, divalproex, diphenhydramine, doxepin, estazolam, ethchlorvynol, etomidate, fenobam, flunitrazepam, flurazepam, fluvoxamine, fluoxetine, fosazepam, glutethimide, halazepam, hydroxyzine, imipramine, lithium, lorazepam, lormetazepam, maprotiline, mecloqualone, melatonin, mephobarbital, meprobamate, methaqualone, midaflur, midazolam, nefazodone, nisobamate, nitrazepam, nortriptyline, oxazepam, paraldehyde, paroxetine, pentobarbital, perlapine, perphenazine, phenelzine, phenobarbital, prazepam, promethazine, propofol, protriptyline, quazepam, reclazepam, roletamide, secobarbital, sertraline, suproclone, temazepam, thioridazine, tracazolate, tranylcypromaine, trazodone, triazolam, trepipam, tricetamide, triclofos, trifluoperazine, trimetozine, trimipramine, uldazepam, venlafaxine, zaleplon, zolazepam, zolpidem, and salts thereof, and combinations thereof, and the like, or the subject compound may be administered in conjunction with the use of physical methods such as with light therapy or electrical stimulation.

In another embodiment, the subject compound may be employed in combination with levodopa (with or without a selective extracerebral decarboxylase inhibitor such as carbidopa or benserazide), anticholinergics such as biperiden (optionally as its hydrochloride or lactate salt) and trihexyphenidyl (benzhexol) hydrochloride, COMT inhibitors such as entacapone, MOA-B inhibitors, antioxidants, A2a adenosine receptor antagonists, cholinergic agonists, NMDA receptor antagonists, serotonin receptor antagonists and dopamine receptor agonists such as alentemol, bromocriptine, fenoldopam, lisuride, naxagolide, pergolide and pramipexole. It will be appreciated that the dopamine agonist may be in the form of a pharmaceutically acceptable salt, for example, alentemol hydrobromide, bromocriptine mesylate, fenoldopam mesylate, naxagolide hydrochloride and pergolide mesylate. Lisuride and pramipexol are commonly used in a non-salt form.

In another embodiment, the subject compound may be employed in combination with acetophenazine, alentemol, benzhexol, bromocriptine, biperiden, chlorpromazine, chlorprothixene, clozapine, diazepam, fenoldopam, fluphenazine, haloperidol, levodopa, levodopa with benserazide, levodopa with carbidopa, lisuride, loxapine, mesoridazine, molindolone, naxagolide, olanzapine, pergolide, perphenazine, pimozide, pramipexole, risperidone, sulpiride, tetrabenazine, trihexyphenidyl, thioridazine, thiothixene or trifluoperazine.

In another embodiment, the subject compound may be employed in combination with a compound from the phenothiazine, thioxanthene, heterocyclic dibenzazepine, butyrophenone, diphenylbutylpiperidine and indolone classes of neuroleptic agent. Suitable examples of phenothiazines include chlorpromazine, mesoridazine, thioridazine, acetophenazine, fluphenazine, perphenazine and trifluoperazine. Suitable examples of thioxanthenes include chlorprothixene and thiothixene. An example of a dibenzazepine is clozapine. An example of a butyrophenone is haloperidol. An example of a diphenylbutylpiperidine is pimozide. An example of an indolone is molindolone. Other neuroleptic agents include loxapine, sulpiride and risperidone. It will be appreciated that the neuroleptic agents when used in combination with the subject compound may be in the form of a pharmaceutically acceptable salt, for example, chlorpromazine hydrochloride, mesoridazine besylate, thioridazine hydrochloride, acetophenazine maleate, fluphenazine hydrochloride, flurphenazine enathate, fluphenazine decanoate, trifluoperazine hydrochloride, thiothixene hydrochloride, haloperidol decanoate, loxapine succinate and molindone hydrochloride. Perphenazine, chlorprothixene, clozapine, haloperidol, pimozide and risperidone are commonly used in a non-salt form.

In another embodiment, the subj ect compound may be employed in combination with an anoretic agent such as aminorex, amphechloral, amphetamine, benzphetamine, chlorphentermine, clobenzorex, cloforex, clominorex, clortermine, cyclexedrine, dexfenfluramine, dextroamphetamine, diethylpropion, diphemethoxidine, N-ethylamphetamine, fenbutrazate, fenfluramine, fenisorex, fenproporex, fludorex, fluminorex, furfurylmethylamphetamine, levamfetamine, levophacetoperane, mazindol, mefenorex, metamfepramone, methamphetamine, norpseudoephedrine, pentorex, phendimetrazine, phenmetrazine, phentermine, phenylpropanolamine, picilorex and sibutramine; selective serotonin reuptake inhibitor (SSRI); halogenated amphetamine derivatives, including chlorphentermine, cloforex, clortermine, dexfenfluramine, fenfluramine, picilorex and sibutramine; and pharmaceutically acceptble salts thereof

In another embodiment, the subject compound may be employed in combination with an opiate agonist, a lipoxygenase inhibitor, such as an inhibitor of 5-lipoxygenase, a cyclooxygenase inhibitor, such as a cyclooxygenase-2 inhibitor, an interleukin inhibitor, such as an interleukin-1 inhibitor, an NMDA antagonist, an inhibitor of nitric oxide or an inhibitor of the synthesis of nitric oxide, a non-steroidal antiinflammatory agent, or a cytokine-suppressing antiinflammatory agent, for example with a compound such as acetaminophen, asprin, codiene, fentanyl, ibuprofen, indomethacin, ketorolac, morphine, naproxen, phenacetin, piroxicam, a steroidal analgesic, sufentanyl, sunlindac, tenidap, and the like. Similarly, the subject compound may be administered with a pain reliever; a potentiator such as caffeine, an H2-antagonist, simethicone, aluminum or magnesium hydroxide; a decongestant such as phenylephrine, phenylpropanolamine, pseudophedrine, oxymetazoline, ephinephrine, naphazoline, xylometazoline, propylhexedrine, or levo-desoxy-ephedrine; an antiitussive such as codeine, hydrocodone, caramiphen, carbetapentane, or dextramethorphan; a diuretic; and a sedating or non-sedating antihistamine.

The compounds of the present invention may be administered by oral, parenteral (e.g., intramuscular, intraperitoneal, intravenous, ICV, intracisternal injection or infusion, subcutaneous injection, or implant), by inhalation spray, nasal, vaginal, rectal, sublingual, or topical routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals such as mice, rats, horses, cattle, sheep, dogs, cats, monkeys, etc., the compounds of the invention are effective for use in humans.

The pharmaceutical compositions for the administration of the compounds of this invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active object compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

Pharmaceutical compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. Compositions for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil. Aqueous suspensions contain the active materials in admixture with excipients suitable for the manufacture of aqueous suspensions. Oily suspensions may be formulated by suspending the active ingredient in a suitable oil. Oil-in-water emulsions may also be employed. Dispersible powders and granules suitable for preparation of an aqueous suspension by the addition of water provide the active ingredient in admixture with a dispersing or wetting agent, suspending agent and one or more preservatives. Pharmaceutical compositions of the present compounds may be in the form of a sterile injectable aqueous or oleagenous suspension. The compounds of the present invention may also be administered in the form of suppositories for rectal administration. For topical use, creams, ointments, jellies, solutions or suspensions, etc., containing the compounds of the present invention may be employed. The compounds of the present invention may also be formulated for administered by inhalation. The compounds of the present invention may also be administered by a transdermal patch by methods known in the art.

Several methods for preparing the compounds of this invention are illustrated in the following Schemes and Examples. Starting materials are made according to procedures known in the art or as illustrated herein. The following abbreviations are used herein: Me: methyl; Et: ethyl; t-Bu: *tert*-butyl; Ar: aryl; Ph: phenyl; Bn: benzyl; Ac: acetyl; THF: tetrahydrofuran; DEAD: diethylazodicarboxylate; DIPEA: N,N-diisopropylethylamine; DMSO: dimethylsulfoxide; EDC: N-(3-Dimethylaminopropyl)-N'-ethylcarbodiimide; HOBT: hydroxybenzotriazole hydrate; Boc: tert-butyloxy carbonyl; Et₃N: triethylamine; DCM: dichloromethane; DCE: dichloroethane; BSA: bovine serum albumin; TFA: trifluoracetic acid; DMF: N,N-dimethylformamide; MTBE: methyl tert-butyl ether;SOCl₂: thionyl chloride; CDI: carbonyl diimidazole; rt: room temperature; HPLC: high performance liquid chromatography. The compounds of the present invention can be prepared in a variety of fashions.

In some cases the final product may be further modified, for example, by manipulation of substituents. These manipulations may include, but are not limited to, reduction, oxidation, alkylation, acylation, and hydrolysis reactions which are commonly known to those skilled in the art. In some cases the order of carrying out the foregoing reaction schemes may be varied to facilitate the reaction or to avoid unwanted reaction products. The following examples are provided so that the invention might be more fully understood. These examples are illustrative only and should not be construed as limiting the invention in any way.

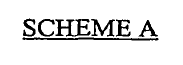

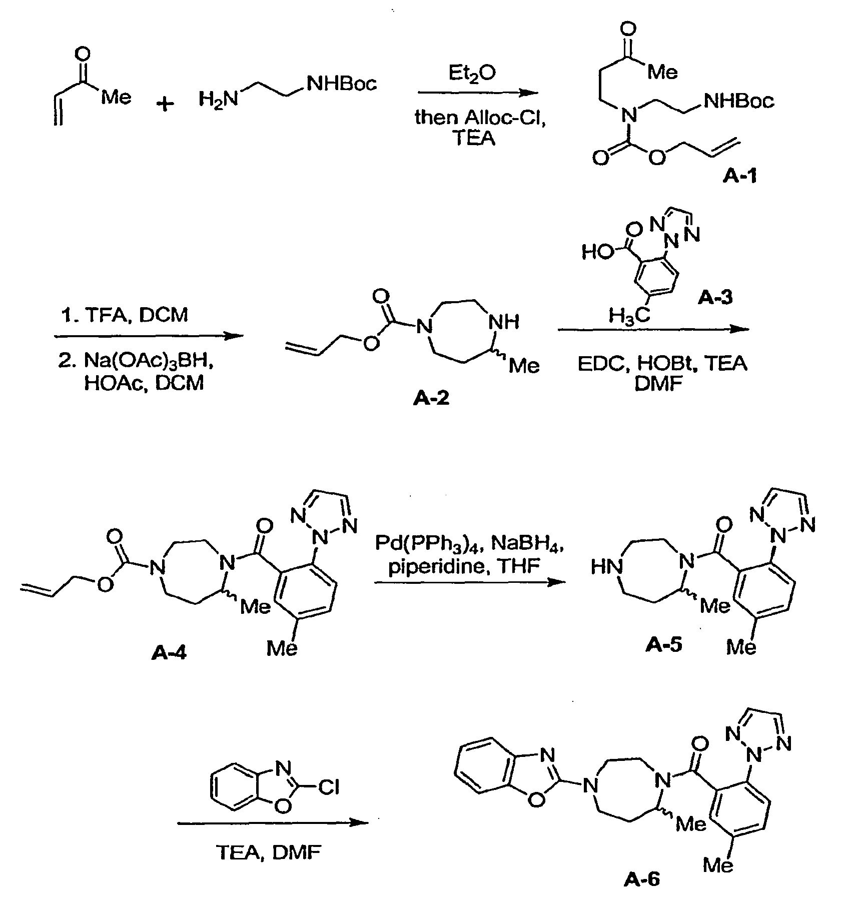

### methyl ketone A-1

A solution of Boc-ethylenediamine (5.7 g, 35.7 mmol) in 100 mL Et₂O was treated dropwise with 2.92 mL (35.7 mmol) methyl vinyl ketone and was allowed to stir 24 h. The reaction was then cooled to 0° and 6.21 mL (44.6 mmol) triethylamine was added, followed by 4.16 mL (39.2 mmol) allyl chloroformate. The reaction was allowed to slowly warm to room temperature with stirring overnight. The reaction was diluted with EtOAc, washed with an aqueous 10% citric acid solution, then with saturated NaHCO₃, and then brine. The organic phase was dried over Na₂SO₄, filtered and concentrated to provide A-1 as a pale yellow oil. Data for A-1: ¹HNMR (500 MHz, CDCl₃) δ 5.95 (m, 1H), 5.35 - 5.2 (m, 2H), 5.0 - 4.6 (m, 1H), 4.6 (m, 2H), 3.5 (m, 2H), 3.4 (m, 2H), 3.0 (m, 2H), 2.5 (m, 2H), 2.15 (s, 3H), 1.5 (s, 9H) ppm.

### allyl 5-methyl-1,4-diazepane-1-carboxylate (A-2)

To a solution of 2.9 g (9.2 mmol) A-1 in 50 mL DCM was added 5 mL of TFA. After stirring for 4 h, the solvents were removed by rotary evaporation, followed by an azeotrope with toluene. The residue was dissolved in 50 mL of DCM and 1 mL of HOAc. After stirring for 1 h, 3.89 g (18.4 mmol) Na(OAc)₃BH was added and the reaction was stirred at room temperature overnight. The reaction was dumped into a separatory funnel containing a saturated NaHCO₃ solution and 2:1 CHCl₃/EtOH. The layers were separated, and the aqueous was extracted twice more with 2:1 CHCl₃/EtOH. The combined organic layers were washed with a minimum amount of brine and concentrated to provide A-2 as a pale yellow oil. Data for A-2: LC/MS: rt = 0.41 min; *m*/*z* (M + H) = 199.0 found; 199.1 required.

### 2-(2H-1,2,3-triazol-2-yl)-5-methylbenzoic acid (A-3)

A solution of 2-iodo-5-methylbenzoic acid (4.0 g, 15.3 mmol) in DMF (10 mL) was treated with 1,2,3-triazole (2.1 g, 30.5 mmol), CsCO₃ (9.95 g, 30.5 mmol), CuI (0.145 g, 0.76 mmol) and trans-N,N'-dimethylcyclohexane-1,2-diamine (0.43 g, 3.05 mmol). The mixture was heated at 120 °C for 10 min in a microwave reactor. The reaction was cooled to room temperature, diluted with water, and washed with EtOAc. The aqueous phase was acidified with 1N HCl and extracted with EtOAc. The organic layer was dried over Na₂SO₄, filtered and concentrated. The residue was purified by gradient elution on SiO₂ (0 to 10% MeOH in water with 0.1% AcOH) to give the faster eluting 2-(2H-1,2,3-triazol-2-yl)-5-methylbenzoic acid A-3, followed by the undesired regioisomer isomer, 1-(2H-1,2,3-triazol-2-yl)-5-methylbenzoic acid. Data for A-3: ¹HNMR (500 MHz, DMSO-d₆) d 12.98 (br s , 1H), 8.04 (s, 2H), 7.72-7.45 (m, 3H), 2.41 (s, 3H) ppm.

### allyl 5-methyl-4-[5-methyl-2-(2H-1,2,3-triazol-2-yl)benzoyl]-1,4-diazepane-1-carboxylate (A-4)

To a solution of 330 mg (1.66 mmol) A-2, 405 mg (2.0 mmol) A-3, 270 mg (2.0 mmol) 1-hydroxybenzotriazole hydrate, and 700 µL (5.0 mmol) triethylamine in 5 mL of DMF was added 383 mg (2.0 mmol) EDC and the reaction was stirred overnight at 55°C. The reaction was partitioned between EtOAc and saturated aqueous NaHCO₃. The layers were separated and the organic was washed twice with brine, dried over Na₂SO₄ and concentrated by rotary evaporation. The residue was purified by column chromatography on silica gel (EtOAc/hexanes) to provide A-4 as a white taffy. Data for A-4: LC/MS: rt = 2.13 min; *m*/*z* (M + H) = 384.0 found; 384.2 required.

### 7-methyl-1-[5-methyl-2-(2H-1,2,3-triazol-2-yl)benzoyl]-1,4-diazepane (A-5)

A solution of 320 mg (0.84 mmol) A-4 in 10 mL of THF was degassed for 3 minutes with a stream of N₂. To the reaction was then added 330 µL (3.3 mmol) piperidine, 127 mg (3.3 mmol) NaBH₄, and 96 mg (0.08 mmol) tetrakispalladiumtriphenylphosphine. After stirring for 1 h at room temperature, the reaction was dumped into a separatory funnel containing a saturated NaHCO₃ solution and 2:1 CHCl₃/EtOH. The layers were separated, and the aqueous was extracted twice more with 2:1 CHCl₃/EtOH. The combined organic layers were washed with a minimum amount of brine and concentrated to provide crude A-5. Data for A-5: LC/MS: rt = 1.17 min; *m*/*z* (M + H) = 300.1 found; 300.2 required.

### 2-{5-methyl-4-[5-methyl-2-(2H-1,2,3-triazol-2-yl)benzoyl]-1,4-diazepan-1-yl}-1,3-benzoxazole (A-6)

A solution of the crude A-5 from the previous step dissolved in 3 mL DMF was treated with 120 µL (1.1 mmol) 2-chlorobenzoxazole and 350 µL (2.51 mmol) triethylamine and stirred at 55°C for 1.5 h. After cooling to room temperature, the reaction was diluted with EtOAc, washed with saturated aqueous NaHCO₃, then two times with brine and dried over Na₂SO₄. Following concentration by rotary evaporation, the residue was purified by flash column chromatography (hexanes/EtOAc) to provide A-6 as a white solid. Data for A-6: HRMS (APCI) *m*/*z* (M+H) 417.2007 found; 417.2034 required.

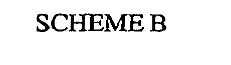

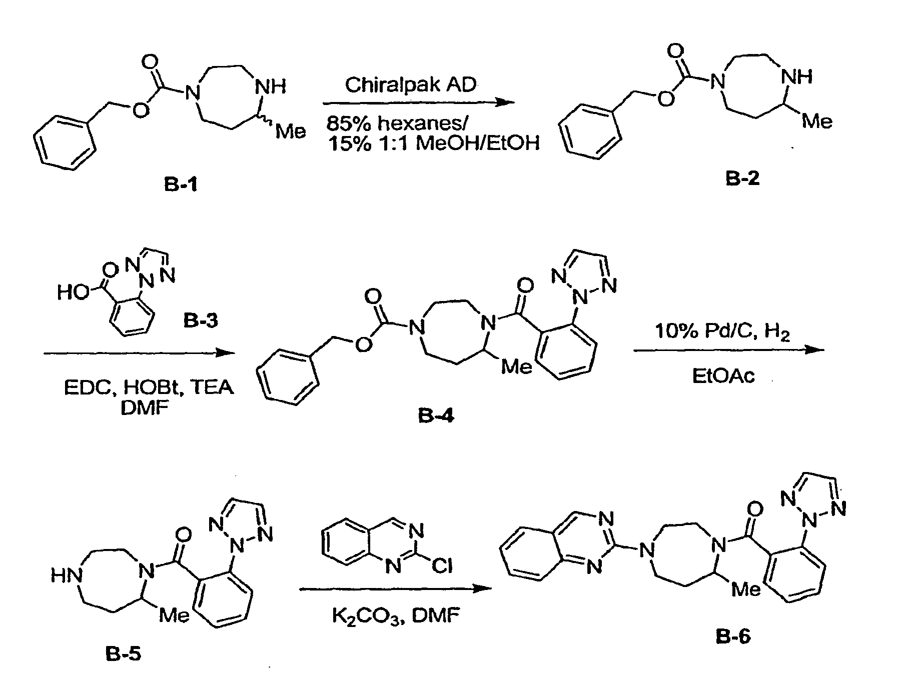

### (R) or (S)-benzyl 5-methyl-1,4-diazepane-1-carboxylate (B-2)

A 3.0 g (12 mmol) racemic mixture of B-1 (prepared by a route analogous to that used for A-2, with the substitution of benzyl chloroformate for allyl chloroformate) was separated into its enatiomers by chrial HPLC utilizing a 10 cm Chiralpak AD column with 15% of 1:1 MeOH/EtOH and 85% hexanes (modified with 0.1% diethylamine) with a flow rate of 200 mL/min. Under these conditions, 1.14 g of the first eluting enantiomer was obtained in > 95% ee, and 1.15 g of the second eluting enantiomer was obtained in > 95% ee. The first eluting enantiomer is designated B-2 and was carried on to provide the desired compounds.

### 2-(2H-1,2,3-triazol-2-yl)benzoic acid B-3

A solution of 2-iodobenzoic acid (3.0 g, 12.09 mmol) in DMF was treated with (1.5 g, 21.7 mmol) 1,2,3-triazole, 7.08 g (21.7 mmol) CsCO₃, 114 mg (0.60 mmol) CuI and 310 mg (2.17 mmol) trans-N,N'-dimethylcyclohexane-1,2-diamine. The mixture was heated at 120 °C for 10 min in a microwave reactor. The reaction was cooled to rt, diluted with EtOAc, and filtered through Celite. The residue was purified by gradient elution on SiO₂ (0 to 10% MeOH in DCM with 0.1% AcOH) to give the faster eluting desired 2-(2H-1,2,3-triazol-2-yl)benzoyl acid, B-3. Data for B-3: ¹HNMR (500 MHz, DMSO-d₆) δ 13.05 (br s , 1H), 8.12 (s, 2H), 7.81-7.52 (m, 4H) ppm. The undesired 1-(2H-1,2,3-triazol-2-yl)benzoic acid eluted second.

### (R) or (S) - benzyl 5-methyl-4-[2-(2H-1,2,3-triazol-2-yl)benzoyl]-1,4-diazepane-1-carboxylate (B-4)

To a solution of 250 mg (1.0 mmol) of the first eluting enantiomer B-2, 200 mg (1.05 mmol) B-3, 185 mg (1.2 mmol) 1-hydroxybenzotriazole hydrate, and 421 µL (3.0 mmol) triethylamine in 3 mL of DMF was added 289 mg (1.5 mmol) EDC and the reaction was stirred 4 h at 50°C. The reaction was partitioned between EtOAc and saturated aqueous NaHCO₃. The layers were separated and the organic was washed with water, brine, dried over MgSO₄ and concentrated by rotary evaporation. The residue was purified by column chromatography on silica gel (EtOAc/hexanes) to provide B-4 as a colorless gum. Data for B-4: LC/MS: rt = 2.25 min; *m*/*z* (M + H) = 420.0 found; 420.2 required.

### (R) or (S)-7-methyl-1-[2-(2H-1,2,3-triazol-2-yl)benzoyl]-1,4-diazepane (B-5)

A solution of 480 mg (1.1 mmol) B-4 in 150 mL EtOAc was evacuated under reduced pressure and purged with N₂ three times before adding a portion of 10% palladium on carbon. After purging three more times with N₂, the atmosphere was replaced with H₂ and the reaction was stirred under a balloon of H₂ overnight. The reaction was filtered through a pad of celite with rinsing by EtOAc and MeOH, and the filtrate was concentrated to provide crude B-5 as a white solid. Data for B-5: LC/MS: rt = 1.02 min; *m*/*z* (M + H) = 286.0 found; 286.2 required.

### (R) or (S)-2-{5-methyl-4-[2-(2H-1,2,3-triazol-2-yl)benzoyl]-14-diazepan-1-yl}quinazoline (B-6)

A solution of 50 mg (0.18 mmol) B-5 in 3 mL DMF was treated with 32 mg (0.19 mmol) 2-chloroquinazoline (prepared according to US 6,040,448) and 121 mg (0.88 mmol) potassium carbonate and stirred at 50°C overnight. After cooling to room temperature, the reaction was diluted with EtOAc, washed with saturated aqueous NaHCO₃, then with water, brine and dried over MgSO₄. Following concentration by rotary evaporation, the residue was purified by flash column chromatography (CHCl₃/MeOH), followed by a second chromatography (EtOAc/hexanes) to provide A-6 as a pale yellow solid. Data for B-6: HRMS (APCI) *m*/*z* (M+H) 414.2015 found; 414.2037 required.

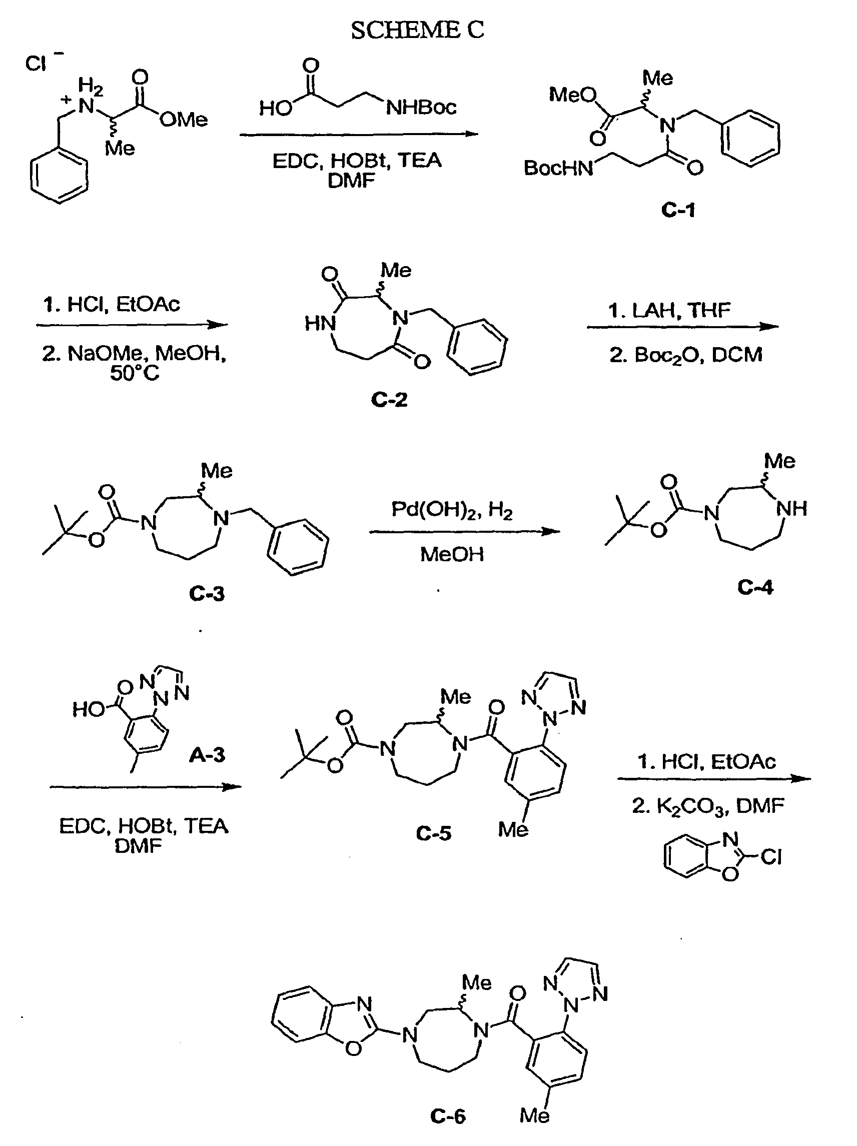

### methyl N-(tert-butoxycarbonyl)-β-alanyl-N-benzylalaninate (C-1)

To a solution of 5.4 g (23.5 mmol) Bzl-Ala-OMe HCl, 5.34 g (28.2 mmol) Boc-β-alanine, 3.97 g (29.4 mmol) 1-hydroxybenzotriazole hydrate, and 9.83 mL (70.5 mmol) triethylamine in 30 mL of DMF was added 5.4 g (28.2 mmol) EDC and the reaction was stirred 4 h at room temperature. The reaction was partitioned between EtOAc and 10% aqueous citric acid, the layers were separated and the organic was washed with 5% aqueous Na₂CO₃, then with brine, dried over Na₂SO₄ and concentrated by rotary evaporation. The residue was purified by column chromatography on silica gel (EtOAc/hexanes) to provide C-1 as a colorless oil. Data for C-1: LC/MS: rt = 222 min; *m*/*z* (M + H) = 265.1 found; 265.2 required for [M-Boc]⁺.

### 4-benzyl-3-methyl-1,4-diazepane-2,5-dione (C-2)

A solution of 1.2 g (3.3 mmol) C-1 in 50 mL EtOAc was treated with HCl (g) until the solution was warm to the touch. The reaction flask was sealed with a stopper and allowed to stir at room temperature overnight. The solvents were removed by rotary evaporation, and the residue was basified with saturated aqueous NaHCO₃, and extracted with three portions of 2:1 CHCl₃/EtOH. The organic exytracts were concentrated to 650 mg of a pale yellow gum. This material was dissolved in 10 mL of dry MeOH and treated with 159 mg (2.95 mmol) NaOMe and the resultant mixture was stirred at 60°C overnight. The reaction was cooled to room temperature and quenched with aqueous NH₄Cl. The reaction was then dumped into a separatory funnel containing 5% aqueous Na₂CO₃ and extracted with three portions of 2:1 CHCl₃/EtOH. The organic layers were combined and concentrated to provide C-2 as a white taffy. Data for C-2: LC/MS: rt = 1.17 min; *m*/*z* (M+H)= 233.1, found; 233.1 required.

### tert-butyl 4-benzyl-3-methyl-1,4-diazepane-1-carboxylate (C-3)

A solution of 1.37 g (5.9 mmol) C-2 in 60 mL THF at 0°C was treated with 35.4 mL (35.4 mmol) of a 1M solution of LAH in THF. The reaction was slowly warmed to room temperature with stirring and allowed to stir at room temperature for 72 h. The reaction was then cooled to -10°C and was carefully quenched with 1.5 mL water, then 1.5 mL 15% NaOH, followed by an additional 4.5 mL of water. A portion of Na₂SO₄ was added and the mixture was stirred for 1 h before being filtered through celite. The filtrate was concentrated to provide 1.2 g of a colorless oil. This material was dissolved in 30 mL of DCM and 1.54 g (7.1 mmol) of Boc₂O was added and the reaction was stirred for 2 h at room temperature. The reaction was concentrated and loaded directly onto a column and the residue was purified by flash column chromatography (EtOAc/hexanes) to provide C-3 as a colorless oil. Data for C-3: LC/MS: rt = 1.34 min; *m*/*z* (M + H) = 305.1, found; 305.2 required.

### tert-butyl 3-methyl-1,4-diazepane-1-carboxylate (C-4)

A solution of 1.58 g (5.19 mmol) C-3 in 20 mL MeOH was evacuated under reduced pressure and purged with N₂ before adding a portion of Pd(OH)₂. After purging two more times with N₂, the atmosphere was replaced with H₂ and the reaction was stirred under a balloon of H₂ for 2 h. The reaction was filtered through a pad of celite and the filtrate was concentrated to provide C-4 as a pale yellow oil. Data for C-4: LC/MS: rt = 1.03 min; *m*/*z* (M + H) = 215.2 found; 215.2 required.

### tert-butyl 3-methyl-4-[5-methyl-2-(2H-1,2,3-triazol-2-yl)benzoyl]-1,4-diazepane-1-carboxylate (C-5)

To a solution of 420 mg (1.96 mmol) C-4, 478 mg (2.35 mmol) A-3, 318 mg (2.35 mmol) 1-hydroxybenzotriazole hydrate, and 820 µL (5.9 mmol) triethylamine in 3 mL of DMF was added 451 mg (2.35 mmol) EDC and the reaction was stirred 2 h at 50°C. The reaction was partitioned between EtOAc and saturated aqueous NaHCO₃ The layers were separated and the organic was washed twice with brine, dried over Na₂SO₄ and concentrated by rotary evaporation. The residue was purified by column chromatography on silica gel (EtOAc/hexanes) to provide C-5 as a white taffy. Data for C-5: LC/MS: rt = 2.24 min; *m*/*z* (M + H) = 400.0, found; 400.2 required.

### 2-{3-methyl-4-[5-methyl-2-(2H-1,2,3-triazol-2-yl)benzoyl]-1,4-diazepan-1-yl}-1,3-benzoxazole (C-6)

A solution of 550 mg (1.38 mmol) C-5 in 20 mL EtOAc was treated with HCl (g) until the solution was warm to the touch. The reaction flask was sealed with a stopper and allowed to stir at room temperature for 2 h. The solvents were removed by rotary evaporation, the residue was triturated with Et₂O, and again concentrated to provide 510 mg of a white solid. To 114 mg (0.34 mmol) of this material in 3 mL of DMF was added 39 mL (0.34 mmol) 2-chlorobenzoxazole and 142 mg (1.0 mmol) potassium carbonate and the reaction was heated at 50°C overnight. After cooling to room temperature, the reaction was diluted with EtOAc, washed with saturated aqueous NaHCO₃, then 3 times with brine and dried over Na₂SO₄. Following concentration by rotary evaporation, the residue was purified by flash column chromatography (hexanes/EtOAc) to provide C-6 as a white taffy. Data for C-6: HRMS (APCI) *mlz* (M+H) 417.2001 found; 417.2034 required.

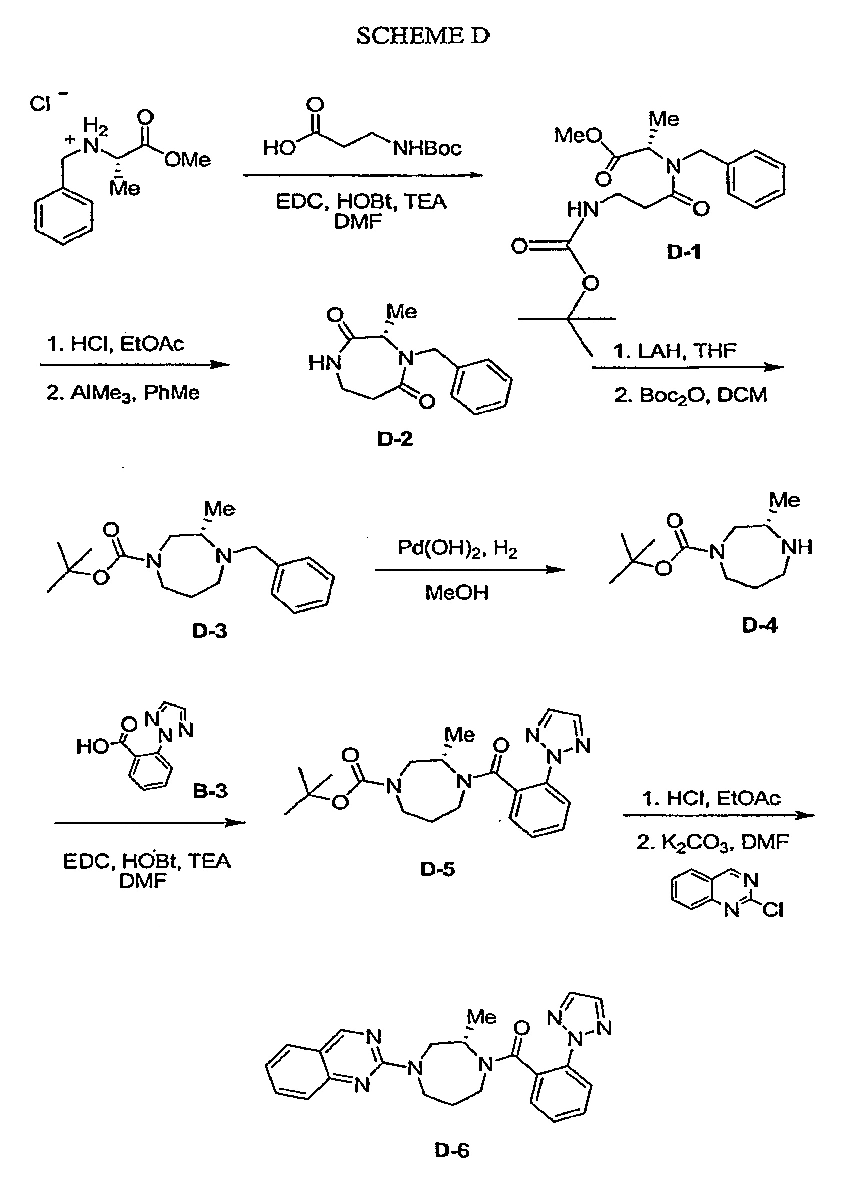

### (L) - methyl N-(tert-butoxycarbonyl)-β-alanyl-N-benzylalaninate (D-1)

To a solution of 10.0 g (43.5 mmol) L-Bzl-Ala-OMe HCl, 8.65 g (45.7 mmol) Boc-β-alanine, 8.0 g (52.2 mmol) 1-hydroxybenzotriazole hydrate, and 18.2 mL (131 mmol) triethylamine in 100 mL of DMF was added 12.52 g (65.3 mmol) EDC and the reaction was stirred overnight at 50°C. The reaction was partitioned between EtOAc and 10% aqueous KHSO₄, the layers were separated and the organic was washed with saturated aqueous NaHCO₃, then with water, brine, dried over MgSO₄ and concentrated by rotary evaporation. The residue was purified by column chromatography on silica gel (EtOAc/hexanes) to provide D-1 as a colorless gum. Data for C-1: LC/MS: rt = 2.22 min; *mlz* (M + H) = 265.1 found; 265.2 required for [M - Boc]⁺.

### (S)-4-benzyl-3-methyl-1,4-diazepane-2,5-dione (D-2)

A solution of 9.8 g (26.9 mmol) D-1 in 100 mL EtOAc was saturated with HCl (g), stirred for 15 minutes, then saturated again. The reaction flask was sealed with a stopper and allowed to stir at room temperature for 30 minutes. The solvents were removed by rotary evaporation, and the residue was dissolved in water and basified basified K₂CO₃. The solution was extracted twice with EtOAc, the combined organic extracts were washed with brine, dried over MgSO4, and concentrated to provide 5.43 g of an amber gum. This material was dissolved in 80 mL of dry toluene, cooled to 0°C, and treated over 90 minutes by dropwise addition of 20.5 mL (41.1 mmol) of a 2.0 M solution of AlMe₃ in toluene. The mixture is allowed to warm to room temperature and stir for 5 h, at which time it was quenched with ∼ 100 mL of 1M HCl and partitioned with EtOAc. After separation of the layers, the aqueous is again extracted with EtOAc, the combined organic layers are washed with water, brine, dried over MgSO₄, and concentrated to provide D-2 as an amber gum. Data for D-2: LC/MS: rt = 1.15 min; *mlz* (M + H) = 233.1, found; 233.1 required.

### (S)-tert-butyl 4-benzyl-3-methyl-1,4-diazepane-1-carboxylate (D-3)

A solution of 5.15 g (22.2 mmol) D-2 in 100 mL THF at 0°C was treated with 55.5 mL (222 mmol) of a 4M solution of LAH in Et₂O. The reaction was slowly warmed to room temperature with stirring and allowed to stir at room temperature overnight. The reaction was then cooled to 0°C and was carefully quenched with 8.5 mL water, then 8.5 mL 15% NaOH, followed by an additional 25.5 mL of water. A portion of THF (∼ 300 mL) was added and the mixture was stirred for 15 minutes before being filtered through celite. The filtrate was concentrated to provide 4.13 g of an orange gum. This material was dissolved in 50 mL of DCM and 5.63 mL (40.4 mmol) triethylamine and 6.62 g (30.3 mmol) of Boc₂O were added and the reaction was stirred overnight at room temperature. The reaction was partitioned between EtOAc and saturated aqueous NaHCO₃. The layers were separated and the organic was washed with water, brine, dried over MgSO₄ and concentrated by rotary evaporation. The residue was purified by flash column chromatography (EtOAc/hexanes) to provide D-3 as a colorless gum. Data for C-3: LC/MS: rt = 1.33 min; *m*/*z* (M + H) = 305.1, found; 305.2 required.

### (S) - tert-butyl 3-methyl-1,4-diazepane-1-carboxylate (D-4)

A solution of 1.25 g (5.19 mmol) D-4 in a 1:1 MeOH/EtOAc was evacuated under reduced pressure and purged with N₂ three times before adding a portion of Pd(OH)₂. After purging three more times with N₂, the atmosphere was replaced with H₂ and the reaction was stirred under a balloon of H₂ overnight. The reaction was filtered through a pad of celite and the filtrate was concentrated to provide D-4 as a gum. Data for D-4: LC/MS: rt = 1.0 min; *m*/*z* (M + H) = 215.2 found; 215.2 required.

### (S) - tert-butyl 3-methyl-4-[5-methyl-2-(2H-1,2,3-triazol-2-yl)benzoyl]-1,4-diazepane-1-carboxylate (D-5)

To a solution of 400 mg (1.87 mmol) D-4, 371 mg (1.96 mmol) B-3, 343 mg (2.24 mmol) 1-hydroxybenzotriazole hydrate, and 780 µL (5.6 mmol) triethylamine in 5 mL of DMF was added 537 mg (2.8 mmol) EDC and the reaction was stirred overnight at 50°C. The reaction was partitioned between EtOAc and saturated aqueous NaHCO₃. The layers were separated and the organic was washed with water, brine, dried over MgSO₄ and concentrated by rotary evaporation. The residue was purified by column chromatography on silica gel (EtOAc/hexanes) to provide D-5 as a colorless gum. Data for D-5: LC/MS: rt = 2.09 min; m/z (M + H) = 386.1, found; 386.2 required.

### (S)-2-{3-methyl-4-[5-methyl-2-(2H-1,2,3-triazol-2-yl)benzoyl]-1,4-diazepan-1-yl}-1,3-benzoxazole (C-6)

A solution of 660 mg (1.71 mmol) D-5 in 75 mL EtOAc was saturated with HCl (g), stirred for 15 minutes, then saturated again. The reaction flask was sealed with a stopper and allowed to stir at room temperature for 30 minutes. The solvents were removed by rotary evaporation to provide 500 mg of a fluffy white solid. To 250 mg (0.78 mmol) of this material in 5 mL of DMF was added 141 mg (0.86 mmol) 2-chloroquinazoline (prepared according to US 6,040,448) and 429 mg (3.11 mmol) potassium carbonate and the reaction was heated at 50°C for 4 h and then stirred at room temperature over the weekend. After cooling to room temperature, the reaction was diluted with EtOAc, washed with saturated aqueous NaHCO₃, then with water, brine and dried over MgSO₄. Following concentration by rotary evaporation, the residue was purified by flash column chromatography (hexanes/EtOAc) to provide D-6 as a yellow solid. Data for D-6: HRMS (APCI) *mlz* (M+H) 414.2017 found; 414.2037 required. Comparison of this material to a racemic sample prepared by the method illustrated in Scheme C indicated that D-6 was ~ 93% ee. Conditions: Chiralpak AD with 40% IPA/hexanes (containing 0.1% diethylamine) at 1 mL/min. Racemic material: Rt₍ₚₑₐₖ₁₎ = 6.8 min; Rt₍ₚₑₐₖ₂₎ = 8.6 min. D-6: Rt = 6.8 min.

**TABLE 1**

| | | | |
|---|---|---|---|
| The following compounds were prepared using the foregoing methodology, but substituting the appropriately substituted reagent, as described in the foregoing Reaction Schemes and Examples. The requisite starting materials were commercially available, described in the literature or readily synthesized by one skilled in the art of organic synthesis without undue experimentation. Some final products were purified by flash chromatography (SiO₂; EtOAc/hexanes) and were isolated as the free-base; alternately, some products were purified by reverse phase HPLC (CH₃CN/H₂O containing 0.1% TFA as a modifier) and isolated as the TFA salt, in which case the masses reported and found are for the free-base. Alternatively, fractions containing the product could be basified with NaHCO₃ and extracted with EtOAc, dried over Na₂SO₄, and concentrated to provide the free-base. | | | |

| Cmp | Structure | Name | HRMS *m*/*z* (M+H) |
|---|---|---|---|
| 1-1 | 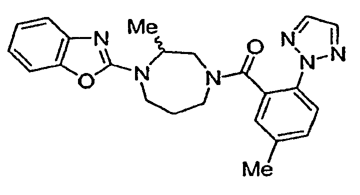 | 2- {2-methyl-4-[5-methyl-2-(2*H*-1,2,3-triazol-2-yl)benzoyl]-1,4-diazepan-1-yl}-1,3-benzoxazole | HRMS *m*/*z* (M+H) 417.2000 found, 417.2034 required. |
| 1-2 | 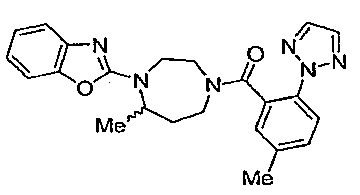 | 2- {7-methyl-4-[5-methyl-2-(2*H*-1,2,3-triazol-2-yl)benzoyl]-1,4-diazepan-1-yl}-1,3-benzoxazole | HRMS *m*/*z* (M+H) 417.2007 found, 417.2034 required. |
| 1-3 | 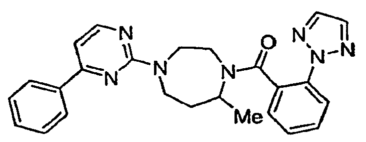 | (*R* or *S*)-5-methyl-1-(4-phenylpyrimidin-2-yl)-4-[2-(2*H*-1,2,3-triazol-2-yl)benzoyl]-1,4-diazepane | HRMS *m*/*z* (M+H) 440.2177 found, 440.2194 required. |
| 1-4 | 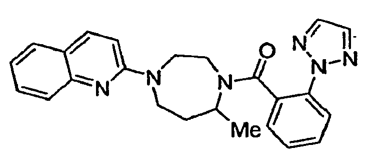 | (*R* or *S*)-2-{5-methyl-4-[2-(2*H*-1,2,3-triazol-2-yl)benzoyl]-1,4-diazepan-1-yl}quinoline | HRMS *mlz* (M+H) 413.2068 found, 413.2085 required. |
| 1-5 | 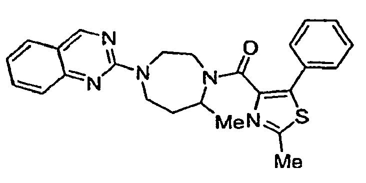 | (*R* or *S*)-2-{5-methyl-4-[(2-methyl-5-phenyl-1,3-thiazol-4-yl)carbonyl]-1,4-diazepan-1-yl} quinazoline | HRMS *m*/*z* (M+H) 444.1837 found, 444.1853 required. |
| 1-6 | 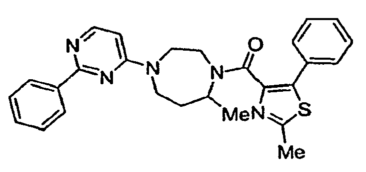 | (*R* or *S*)-5-methyl-4-[(2-methyl-5-phenyl-1,3-thiazol-4-yl)carbonyl]-1-(2-phenylpyrimidin-4-yl)-1,4-diazepane | HRMS *m*/*z* (M+H) 470.1988 found, 470.2009 required. |
| 1-7 | 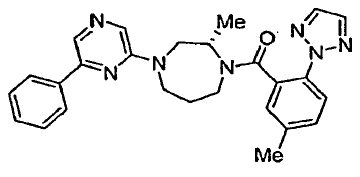 | (2*S*)-2-methyl-1-[5-methyl-2-(2*H*-1,2,3-triazol-2-yl)benzoyl]-4-(6-phenylpyrazin-2-yl)-1,4-diazepane | HRMS *m*/*z* (M+H) 454.2330 found, 454.2350 required. |
| 1-8 | 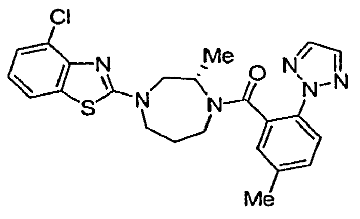 | 4-chloro-2- {(3*S*)-3-methyl-4-[5-methyl-2-(2*H*-1,2,3-triazol-2-yl)benzoyl]-1,4-diazepan-1-yl}-1,3-benzothiazole | HRMS *mlz* (M+H) 467.1395 found, 467.1416 required. |
| 1-9 | 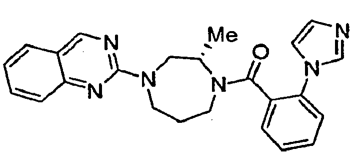 | 2-{(3*S*)-4-[2-(1*H*-imidazol-1-yl)benzoyl]-3-methyl-1,4-diazepan-1-yl}quinazoline | HRMS *m*/*z* (M+H) 413.2062 found, 413.2085required. |
| 1-10 | 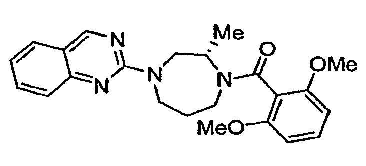 | 2-[(3*S*)-4-(2,6-dimethoxybenzoyl)-3-methyl-1,4-diazepan-1-yl]quinazoline | HRMS *m*/*z* (M+H) 407.2056 found, 407.2078 required. |
| 1-11 | 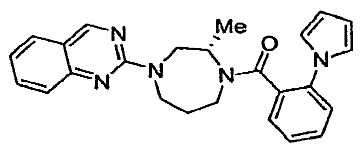 | 2-{(3*S*)-3-methyl-4-[2-(1*H*-pyrrol-1-yl)benzoyl]-1,4-diazepan-1-yl}quinazoline | HRMS *mlz* (M+H) 412.2115 found, 412.2132 required. |
| 1-12 | 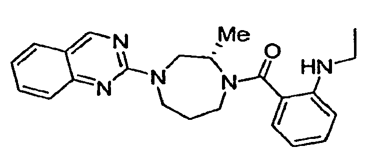 | N-ethyl-2-{[(2*S*)-2-methyl-4-quinazolin-2-yl-1,4-diazepan-1-yl]carbonyl} aniline | HRMS *m*/*z* (M+H) 390.2272 found, 390.2289 required. |
| 1-13 | 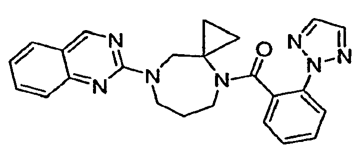 | 2-{4-[2-(2*H*-1,2,3-triazol-2-yl)benzoyl]-4,8-diazaspiro[2.6]non-8-yl}quinazoline | HRMS *m*/*z* (M+H) 426.2008 found, 426.2037 required. |
| 1-14 | 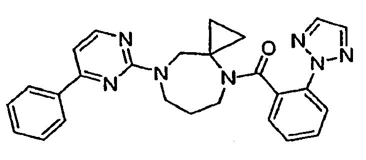 | 8-(4-phenylpyrimidin-2-yl)-4-[2-(2*H*-1,2,3-triazol-2-yl)benzoyl]-4,8-diazaspiro[2.6]nonane | HRMS *m*/*z* (M+H) 452.2165 found, 452.2194 required. |

## Claims

1. A compound of the formula I: 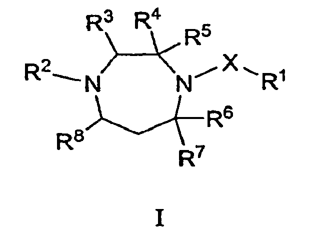 wherein:
X is selected from -SO₂-, -(C=O)-, and -(C=S)-;
R¹ is selected from the group consisting of:
(1) phenyl, where the phenyl is substituted with R^{1a}, R^{1b} and R^{1c}, and
(2) napthyl, where the napthyl is substituted with R^{1a}, R^{1b} and R^{1c};
(3) heteroaryl, where the heteroaryl is substituted with R^{1a}, R^{1b} and R^{1c};
R² is heteroaryl, where the heteroaryl is substituted with R^{2a}, R^{2b} and R^{2c};
R^{1a}, R^{1b}, R^{1c}, R^{2a}, R^{2b} and R^{2c} are independently selected from the group consisting of:
(1) hydrogen,
(2) halogen,
(3) hydroxyl,
(4) -(C=O)ₘ-Oₙ-C₁₋₆alkyl, where m is 0 or 1, n is 0 or 1 (wherein if m is 0 or n is 0, a bond is present) and where the alkyl is unsubstituted or substituted with one or more substituents selected from R¹³,
(5) -(C=O)ₘ-Oₙ-C₃₋₆cycloalkyl, where the cycloalkyl is unsubstituted or substituted with one or more substituents selected from R¹³,
(6) -(C=O)ₘ-C₂₋₄alkenyl, where the alkenyl is unsubstituted or substituted with one or more substituents selected from R¹³,
(7) -(C=O)ₘ-C₂₋₄alkynyl, where the alkynyl is unsubstituted or substituted with one or more substituents selected from R¹³,
(8) -(C=O)ₘ-Oₙ-phenyl or -(C=O)ₘ-Oₙ-napthyl, where the phenyl or napthyl is unsubstituted or substituted with one or more substituents selected from R¹³,
(9) -(C=O)ₘ-Oₙ-heterocycle, where the heterocycle is unsubstituted or substituted with one or more substituents selected from R¹³,
(10) -(C=O)ₘ-NR¹⁰ OR¹¹, wherein R¹⁰ and R¹¹ are independently selected from the group consisting of:
(a) hydrogen,
(b) C₁₋₆alkyl, which is unsubstituted or substituted with one or more substituents selected from R¹³,
(c) C₃₋₆alkenyl, which is unsubstituted or substituted with one or more substituents selected from R¹³,
(d) cycloalkyl, which is unsubstituted or substituted with one or more substituents selected from R¹³,
(e) phenyl, which is unsubstituted or substituted with one or more substituents selected from R¹³, and
(f) heterocycle, which is unsubstituted or substituted with one or more substituents selected from R¹³,
(11) -S(O)₂-NR¹⁰R¹¹,
(12) -S(O)_{q}-R¹², where q is 0, 1 or 2 and where R¹² is selected from the definitions of R10 and R¹¹,
(13) -CO₂H,
(14) -CN,
(15) -NO₂, an
(16) -B(OH)₂;
R³, R⁴, R⁵, R⁶, R⁷ and R⁸ are independently selected from the group consisting of:
(1) - hydrogen,
(2) -C₁₋₆alkyl, where the alkyl is unsubstituted or substituted with one or more substituents selected from R¹³,
(3) -O-C₁₋₆alkyl, where the alkyl is unsubstituted or substituted with one or more substituents selected from R¹³, and
(4) -phenyl, where the phenyl is unsubstituted or substituted with one or more substituents selected from R¹³,
or R⁴ and R⁵, or R⁶ and R⁷ are taken together to form a C₃₋₆cycloalkyl, where the cycloalkyl is unsubstituted or substituted with one or more substituents selected from R¹⁴,
with the proviso that at least one of R³, R⁴, R⁵, R⁶, R⁷ or R⁸ is other than hydrogen;
R¹³ is selected from the group consisting of:
(1) halogen,
(2) hydroxyl,
(3) -(C=O)ₘ-Oₙ-C₁₋₆ alkyl, where the alkyl is unsubstituted or substituted with one or more substituents selected from R¹⁴,
(4) -Oₙ-(C₁₋₃)perfluoroakyl,
(5) -(C=O)ₘ-Oₙ-C₃₋₆cycloalkyl, where the cycloalkyl is unsubstituted or substituted with one or more substituents selected from R¹⁴,
(6) -(C=O)ₘ-C₂₋₄alkenyl, where the alkenyl is unsubstituted or substituted with one or more substituents selected from R14,
(7) -(C=O)ₘ-Oₙ-phenyl or -(C=O)ₘ-Oₙ-napthyl, where the phenyl or napthyl is unsubstituted or substituted with one or more substituents selected from R¹⁴,
(8) -(C=O)ₘ-Oₙ-heterocycle, where the heterocycle is unsubstituted or substituted with one or more substituents selected from R14,
(9) -(C=O)ₘ-NR¹⁰R¹¹,
(10) -S(O)₂-NR¹⁰R¹¹,
(11) -S(O)_{q}-R¹²,
(12) -CO₂H,
(13) -CN, and
(14) -N02;
R14 is selected from the group consisting of:
(1) hydroxyl,
(2) halogen,
(3) C₁₋₆alkyl,
(4) -C₃₋₆cycloalkyl,
(5) -O-C₁₋₆alkyl,
(6) -O(C=O)-C₁₋₆alkyl,
(7) -NH-C₁₋₆alkyl,
(8) phenyl,
(9) heterocycle,
(10) -CO₂H, and
(11) -CN;
or a pharmaceutically acceptable salt thereof.

2. The compound of Claim 1 of the formula la: 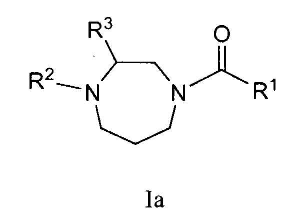 or the formula Ib: 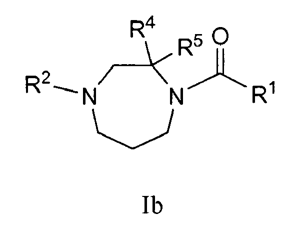 or the formula Ic: 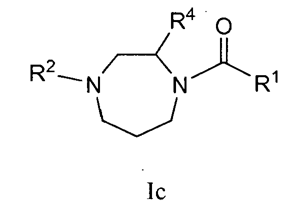 or the formula Ic': 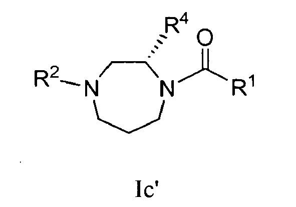 or the formula Id: 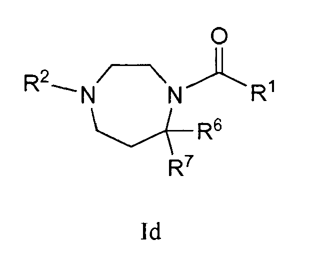 or the formula Ie: 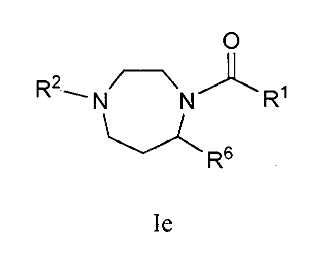 or the formula If: 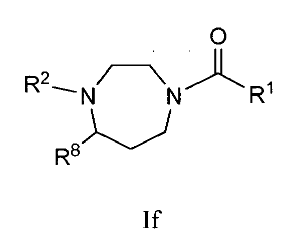 or a pharmaceutically acceptable salt thereof.

3. The compound of Claim 1 wherein X is -(C=O)-.

4. The compound of Claim 1 wherein R¹ is phenyl, which is unsubstituted or substituted with one or more of:
(1) halogen,
(2) hydroxyl,
(3) -Oₙ-C₁₋₆alkyl, where n is 0 or 1 (wherein if n is 0, a bond is present) and where the alkyl is unsubstituted or substituted with one or more substituents selected from R¹³,
(4) -Oₙ-phenyl, where the phenyl is unsubstituted or substituted with one or more substituents selected from R¹³,
(5) -heterocycle, where the heterocycle is unsubstituted or substituted with one or more substituents selected from R¹³,
(6) -NR¹⁰R¹¹, wherein R¹⁰ and R¹¹ are independently selected from the group consisting of:
(a) hydrogen,
(b) C₁₋₆alkyl, which is unsubstituted or substituted with one or more substituents selected from R¹³,
(7) -S(O)₂-NR¹⁰R¹¹,
(8) -CO₂H,
(9) -CN,
(10) -NO₂, and
(11) -B(OH)₂.

5. The compound of Claim 4 wherein R¹ is selected from the group consisting of:
(1) phenyl,
(2) biphenyl,
(3) 2,6-dimethoxyphenyl,
(4) 2,4-dichlorophenyl,
(5) 2,6-dichlorophenyl,
(6) 2,3-difluophenyl,
(7) 2,4-difluophenyl,
(8) 2,6-difluophenyl,
(9) 2-methoxy-4-methyl-phenyl,
(10) 3-methoxy-biphenyl,
(11) 3-methyl-biphenyl, and
(12) 5-methyl-2-triazolyl-phenyl

6. The compound of Claim 1 wherein R² is selected from the group consisting of:
(1) benzimidazolyl,
(2) benzothiazolyl,
(3) benzoxazolyl,
(4) quinazolinyl,
(5) quinolinyl, and
(6) thiadiazolyl,
which is unsubstituted or substituted with halogen, hydroxyl, C₁₋₆alkyl, -O-C₁₋₆alkyl or phenyl.

7. The compound of Claim 6 wherein R² is selected from the group consisting of:
(1) benzimidazol-2-yl,
(2) 1,3-benzothiazol-2-yl,
(3) 1,3-benzoxazol-2-yl,
(4) 2-quinazolinyl,
(5) 1-quinolin-2-yl, and
(6) 1,2,4-thiadiazol-5-yl,
which is unsubstituted or substituted with methyl, fluoro, chloro or phenyl.

8. The compound of Claim 1 wherein R³, R⁴, R⁵, R⁶, R⁷ and R⁸ are independently selected from the group consisting of:
(1) hydrogen, and
(2) -C ₁₋₆alkyl, where the alkyl is unsubstituted or substituted with one or more substituents selected from R¹³,
or R⁴ and R⁵, or R⁶ and R⁷ are taken together to form a C₃₋₆cycloalkyl, where the cycloalkyl is unsubstituted or substituted with one or more substituents selected from R¹⁴,
with the proviso that at least one of R³, R⁴, R⁵, R⁶, R⁷ or R⁸ is other than hydrogen.

9. The compound of Claim 1 wherein one of R³ and R⁴ is -C₁₋₆alkyl, and the remainder of R³-R⁸ are hydrogen.

10. The compound of Claim 1 wherein one of R⁶ and R⁸ is -C₁₋₆alkyl, and the remainder of R³-R⁸ are hydrogen.

11. The compound of Claim 1 wherein R⁴ and R⁵ are taken together to form a C₃₋₆cycloalkyl, or R⁶ and R⁷ are taken together to form a C₃₋₆cycloalkyl, and the remainder of R³-R⁸ arc hydrogen.

12. A compound of Claim 1 which is selected from the group consisting of:
2-{5-methyl-4-[5-methyl-2-(2H-1,2,3-triazol-2-yl)benzoyl]-1,4-diazepan-1-yl}-1,3-benzoxazole;
2-{5-methyl-4-[2-(2H-1,2,3-triazol-2-yl)benzoyl]-1,4-diazepan-1-yl} quinazoline;
2- {3-methyl-4-[5-methyl-2-(2H-1,2,3-triazol-2-yl)benzoyl]-1,4-diazepan-1-yl} -1,3-benzoxazole;
(S)-2-{3-methyl-4-[5-methyl-2-(2H-1,2,3-triazol-2-yl)benzoyl]-1,4-diazepan-1-yl} -1,3-benzoxazole
2-{2-methyl-4-[5-methyl-2-(2H-1,2,3-triazol-2-yl)benzoyl]-1,4-diazepan-1-yl} -1,3-benzoxazole;
2-{7-methyl-4-[5-methyl-2-(2H-1,2,3-triazol-2-yl)benzoyl]-1,4-diazepan-1-yl} -1,3-benzoxazole;
5-methyl-1-(4-phenylpyrimidin-2-yl)-4-[2-(2H-1,2,3-triazol-2-yl)benzoyl]-1,4-diazepane;
2-{5-methyl-4-[2-(2H-1,2,3-triazol-2-yl)benzoyl]-1,4-diazepan-1-yl} quinoline;
2-{5-methyl-4-[(2-methyl-5-phenyl-1,3-thiazol-4-yl)carbonyl]-1,4-diazepan-1-yl } quinazoline;
5-methyl-4-[(2-methyl-5-phenyl-1,3-thiazol-4-yl)carbonyl]-1-(2-phenylpyrimidin-4-yl)-1,4-diazepane;
(2S)-2-methyl-1-[5-methyl-2-(2H-1,2,3-triazol-2-yl)benzoyl]-4-(6-phenylpyrazin-2-yl)-1,4-diazepane;
4-chloro-2-{(3S)-3-methyl-4-[5-methyl-2-(2H-1,2,3-triazol-2-yl)benzoyl]-1,4-diazepan-1-yl}-1,3-benzothiazole;
2-{(3S)-4-[2-(1H-imidazol-1-yl)benzoyl]-3-methyl-1,4-diazepan-1-yl} quinazoline;
2-[(3S)-4-(2,6-dimethoxybenzoyl)-3-methyl-1,4-diazepan-1-yl]quinazoline;
2-{(3S)-3-methyl-4-[2-(1H-pyrrol-1-yl)benzoyl]-1,4-diazepan-1-yl}quinazoline;
N-ethyl-2-{[(2S)-2-methyl-4-quinazolin-2-yl-],4-diazepan-1-yl]carbonyl}aniline;
2-{4-2-(2H-1,2,3-triazol-2-yl)benzoyl]-4,8-diazaspiro[2.6]non-8-yl}quinazoline;
8-(4-phenylpyrimidin-2-yl)-4-[2-(2H-1,2,3-triazol-2-yl)benzoyl]-4,8-diazaspiro[2.6]nonane;
or a pharmaceutically acceptable salt thereof.

13. A pharmaceutical composition which comprises an inert carrier and a compound of any previous claim or a pharmaceutically acceptable salt thereof.

14. A compound according to any of claims 1-12 for use in the treatment or prevention of a sleep disorder.

15. A compound according to any of claims 1-12 for use in treating or controlling obesity in a mammalian patient in need thereof.

## Patentansprüche

1. Eine Verbindung der Formel I: 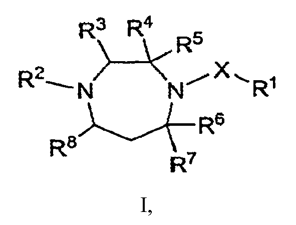 wobei:
X ausgewählt ist aus -SO₂-, -(C=O)- und -(C=S)-,
R¹ ausgewählt ist aus der Gruppe, bestehend aus:
(1) Phenyl, wobei Phenyl substituiert ist mit R^{1a}, R^{1b} und R^{1c}, und
(2) Naphthyl, wobei das Naphthyl substituiert ist mit R^{1a}, R^{1b} und R^{1c},
(3) Heteroaryl, wobei das Heteroaryl substituiert ist mit R^{1a}, R^{1b} und R^{1c},
R² Heteroaryl ist, wobei das Heteroaryl substituiert ist mit R^{2a}, R^{2b} und R^{2c},
R^{1a}, R^{1b}, R^{1c}, R^{2a}, R^{2b} und R^{2c} unabhängig ausgewählt sind aus der Gruppe, bestehend aus:
(1) Wasserstoff,
(2) Halogen,
(3) Hydroxyl,
(4) -(C=O)ₘ-Oₙ-C₁₋₆-Alkyl, wobei m 0 oder 1 ist, n 0 oder 1 ist (wobei, wenn m 0 ist oder n 0 ist, eine Bindung vorliegt) und wobei das Alkyl unsubstituiert oder substituiert ist mit einem oder mehreren Substituenten, ausgewählt aus R¹³,
(5) -(C=O)ₘ-Oₙ-C₃₋₆-Cycloalkyl, wobei das Cycloalkyl unsubstituiert oder substituiert ist mit einem oder mehreren Substituenten, ausgewählt aus R¹³,
(6) -(C=O)ₘ-C₂₋₄-Alkenyl, wobei das Alkenyl unsubstituiert oder substituiert ist mit einem oder mehreren Substituenten, ausgewählt aus R¹³,
(7) -(C=O)ₘ-C₂₋₄-Alkinyl, wobei das Alkinyl unsubstituiert oder substituiert ist mit einem oder mehreren Substituenten, ausgewählt aus R¹³,
(8) -(C=O)ₘ-Oₙ-Phenyl oder -(C=O)ₘ-Oₙ-Naphthyl, wobei das Phenyl oder Naphthyl unsubstituiert oder substituiert ist mit einem oder mehreren Substituenten, ausgewählt aus R¹³,
(9) -(C=O)ₘ-Oₙ-Heterocyclus, wobei der Heterocyclus unsubstituiert oder substituiert ist mit einem oder mehreren Substituenten, ausgewählt aus R¹³,
(10) -(C=O)ₘ-NR¹⁰R¹¹, wobei R¹⁰ und R¹¹ unabhängig ausgewählt sind aus der Gruppe, bestehend aus:
(a) Wasserstoff,
(b) C₁₋₆-Alkyl, unsubstituiert oder substituiert mit einem oder mehreren Substituenten, ausgewählt aus R¹³,
(c) C₃₋₆-Alkenyl, unsubstituiert oder substituiert mit einem oder mehreren Substituenten, ausgewählt aus R¹³,
(d) Cycloalkyl, unsubstituiert oder substituiert mit einem oder mehreren Substituenten, ausgewählt aus R¹³,
(e) Phenyl, unsubstituiert oder substituiert mit einem oder mehreren Substituenten, ausgewählt aus R¹³, und
(f) Heterocyclus, unsubstituiert oder substituiert mit einem oder mehreren Substituenten, ausgewählt aus R¹³,
(11) -S(O)₂-NR¹⁰R¹¹,
(12) -S(O)_{q}-R¹², wobei q 0, 1 oder 2 ist und wobei R¹² ausgewählt ist aus den Definitionen von R¹⁰ und R¹¹,
(13) -CO₂H,
(14) -CN,
(15) -NO₂ und
(16) -B(OH)₂,
R³, R⁴, R⁵, R⁶, R⁷ und R⁸ unabhängig ausgewählt sind aus der Gruppe, bestehend aus:
(1) Wasserstoff,
(2) -C₁₋₆-Alkyl, wobei das Alkyl unsubstituiert oder substituiert ist mit einem oder mehreren Substituenten, ausgewählt aus R¹³,
(3) -O-C₁₋₆-Alkyl, wobei das Alkyl unsubstituiert oder substituiert ist mit einem oder mehreren Substituenten, ausgewählt aus R¹³, und
(4) -Phenyl, wobei das Phenyl unsubstituiert oder substituiert ist mit einem oder mehreren Substituenten, ausgewählt aus R¹³,
oder R⁴ und R⁵ oder R⁶ und R⁷ zusammengenommen werden zur Bildung eines C₃₋₆-Cycloalkyls, wobei das Cycloalkyl unsubstituiert oder substituiert ist mit einem oder mehreren Substituenten, ausgewählt aus R¹⁴,
mit der Maßgabe, dass wenigstens einer der Reste R³, R⁴, R⁵, R⁶, R⁷ oder R⁸ anders als Wasserstoff ist,
R¹³ ausgewählt ist aus der Gruppe, bestehend aus:
(1) Halogen,
(2) Hydroxyl,
(3) -C(C=O)ₘ-Oₙ-C₁₋₆-Alkyl, wobei das Alkyl unsubstituiert oder substituiert ist mit einem oder mehreren Substituenten, ausgewählt aus R¹⁴,
(4) -Oₙ-(C₁₋₃)-Perfluoralkyl,
(5) -(C=O)ₘ-Oₙ-C₃₋₆-Cycloalkyl, wobei das Cycloalkyl unsubstituiert oder substituiert ist mit einem oder mehreren Substituenten, ausgewählt aus R¹⁴,
(6) -(C=O)ₘ-C₂₋₄-Alkenyl, wobei das Alkenyl unsubstituiert oder substituiert ist mit einem oder mehreren Substituenten, ausgewählt aus R¹⁴,
(7) -(C=O)ₘ-Oₙ-Phenyl oder -(C=O)ₘOₙ-Naphthyl, wobei das Phenyl oder Naphthyl unsubstituiert oder substituiert ist mit einem oder mehreren Substituenten, ausgewählt aus R¹⁴,
(8) -(C=O)ₘ-Oₙ-Heterocyclus, wobei der Heterocyclus unsubstituiert oder substituiert ist mit einem oder mehreren Substituenten, ausgewählt aus R¹⁴,
(9) -(C=O)ₘ-NR¹⁰R¹¹,
(10) -S(O)₂-NR¹⁰R¹¹,
(11) -S(O)_{q}-R¹²,
(12) -CO₂H,
(13) -CN und
(14) -NO₂,
R¹⁴ ausgewählt ist aus der Gruppe, bestehend aus:
(1) Hydroxyl,
(2) Halogen,
(3) C₁₋₆-Alkyl,
(4) -C₃₋₆-Cycloalkyl,
(5) -O-C₁₋₆-Alkyl,
(6) -O(C=O)-C₁₋₆-Alkyl,
(7) -NH-C₁₋₆-Alkyl,
(8) Phenyl,
(9) Heterocyclus,
(10) -CO₂H und
(11) -CN,
oder ein pharmazeutisch annehmbares Salz davon.

2. Die Verbindung gemäß Anspruch 1 der Formel Ia: 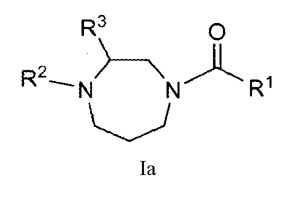 oder der Formel Ib: 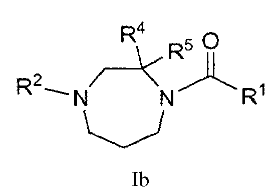 oder der Formel Ic: 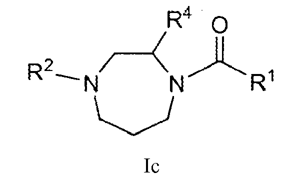 oder der Formel Ic': 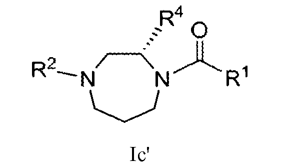 oder der Formel Id: 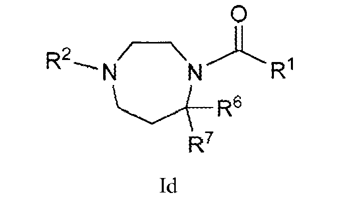 oder der Formel Ie: 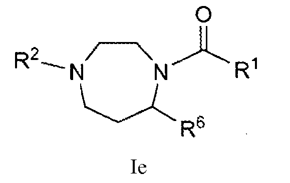 oder der Formel If: 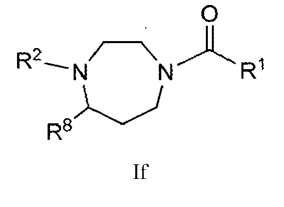 oder ein pharmazeutisch annehmbares Salz davon.

3. Die Verbindung gemäß Anspruch 1, wobei X -(C=O)- ist.

4. Die Verbindung gemäß Anspruch 1, wobei R¹ Phenyl ist, unsubstituiert oder substituiert mit einem oder mehreren:
(1) Halogen,
(2) Hydroxyl,
(3) -Oₙ-C₁₋₆-Alkyl, wobei n 0 oder 1 ist (wobei, wenn n 0 ist, eine Bindung vorliegt) und wobei das Alkyl unsubstituiert oder substituiert ist mit einem oder mehreren Substituenten, ausgewählt aus R¹³,
(4) -Oₙ-Phenyl, wobei das Phenyl unsubstituiert oder substituiert ist mit einem oder mehreren Substituenten, ausgewählt aus R¹³,
(5) -Heterocyclus, wobei der Heterocyclus unsubstituiert oder substituiert ist mit einem oder mehreren Substituenten, ausgewählt aus R¹³,
(6) -NR¹⁰R¹¹, wobei R¹⁰ und R¹¹ unabhängig ausgewählt sind aus der Gruppe, bestehend aus:
(a) Wasserstoff,
(b) C₁₋₆-Alkyl, unsubstituiert oder substituiert mit einem oder mehreren Substituenten, ausgewählt aus R¹³,
(7) -S(O)₂-NR¹⁰R¹¹,
(8) -CO₂H,
(9) -CN,
(10) -NO₂ und
(11) -B(OH)₂.

5. Die Verbindung gemäß Anspruch 4, wobei R¹ ausgewählt ist aus der Gruppe, bestehend aus:
(1) Phenyl,
(2) Biphenyl,
(3) 2,6-Dimethoxyphenyl,
(4) 2,4-Dichlorphenyl,
(5) 2,6-Dichlorphenyl,
(6) 2,3-Difluorphenyl,
(7) 2,4-Difluorphenyl,
(8) 2,6-Difluorphenyl,
(9) 2-Methoxy-4-methylphenyl,
(10) 3-Methoxybiphenyl,
(11) 3-Methylbiphenyl und
(12) 5-Methyl-2-triazolylphenyl.

6. Die Verbindung gemäß Anspruch 1, wobei R² ausgewählt ist aus der Gruppe, bestehend aus:
(1) Benzimidazolyl,
(2) Benzothiazolyl,
(3) Benzoxazolyl,
(4) Chinazolinyl,
(5) Chinolinyl und
(6) Thiadiazolyl,
unsubstituiert oder substituiert mit Halogen, Hydroxyl, C₁₋₆-Alkyl, -O-C₁₋₆-Alkyl oder Phenyl.

7. Die Verbindung gemäß Anspruch 6, wobei R² ausgewählt ist aus der Gruppe, bestehend aus:
(1) Benzimidazol-2-yl,
(2) 1,3-Benzothiazol-2-yl,
(3) 1,3-Benzoxazol-2-yl,
(4) 2-Chinazolinyl,
(5) 1-Chinolin-2-yl und
(6) 1,2,4-Thiadiazol-5-yl,
unsubstituiert oder substituiert mit Methyl, Fluor, Chlor oder Phenyl.

8. Die Verbindung gemäß Anspruch 1, wobei R³, R⁴, R⁵, R⁶, R⁷ und R⁸ unabhängig ausgewählt sind aus der Gruppe, bestehend aus:
(1) Wasserstoff und
(2) -C₁₋₆-Alkyl, wobei das Alkyl unsubstituiert oder substituiert ist mit einem oder mehreren Substituenten, ausgewählt aus R¹³,
oder R⁴ und R⁵ oder R⁶ und R⁷ zusammengenommen werden zur Bildung eines C₃₋₆-Cycloalkyls, wobei das Cycloalkyl unsubstituiert oder substituiert ist mit einem oder mehreren Substituenten, ausgewählt aus R¹⁴,
mit der Maßgabe, dass wenigstens einer der Reste R³, R⁴, R⁵, R⁶, R⁷ oder R⁸ anders als Wasserstoff ist.

9. Die Verbindung gemäß Anspruch 1, wobei einer der Reste R³ und R⁴ -C₁₋₆-Alkyl ist und der Rest von R³-R⁸ Wasserstoff sind.

10. Die Verbindung gemäß Anspruch 1, wobei einer der Reste R⁶ und R⁸ -C₁₋₆-Alkyl ist und der Rest von R³-R⁸ Wasserstoff sind.

11. Die Verbindung gemäß Anspruch 1, wobei R⁴ und R⁵ zusammengenommen werden zur Bildung eines C₃₋₆-Cyclalkyls, oder R⁶ und R⁷ zusammengenommen werden zur Bildung eines C₃₋₆-Cycloalkyls, und der Rest von R³-R⁸ Wasserstoff sind.

12. Eine Verbindung gemäß Anspruch 1, ausgewählt aus der Gruppe, bestehend aus:
2-{5-Methyl-4-[5-methyl-2-(2H-1,2,3-triazol-2-yl)benzoyl]-1,4-diazepan-1-yl}-1,3-benzoxazol, 2-{5-Methyl-4-[2-(2H-1,2,3-triazol-2-yl)benzoyl]-1,4-diazepan-1-yl} chinazolin,
2-{3-Methyl-4-[5-methyl-2-(2H-1,2,3-triazol-2-yl)benzoyl]-1,4-diazepan-1-yl}-1,3-benzoxazol,
(S)-2-{3-Methyl-4-[5-methyl-2-(2H-1,2,3-triazol-2-yl)benzoyl]-1,4-diazepan-1-yl}-1,3-benzoxazol,
2-{2-Methyl-4-[5-methyl-2-(2H-1,2,3-triazol-2-yl)benzoyl]-1,4-diazepan-1-yl}-1,3-benzoxazol,
2-{7-Methyl-4-[5-methyl-2-(2H-1,2,3-triazol-2-yl)benzoyl]-1,4-diazepan-1-yl}-1,3-benzoxazol,
5-Methyl-1-(4-phenylpyrimidin-2-yl)-4-[2-(2H-1,2,3-triazol-2-yl)benzoyl]-1,4-diazepan,
2-{5-Methyl-4-[2-(2H-1,2,3-triazol-2-yl)benzoyl]-1,4-diazepan-1-yl} chinolin,
2-{5-Methyl-4-[(2-methyl-5-phenyl-1,3-thiazol-4-yl)carbonyl]-1,4-diazepan-1-yl} chinazolin,
5-Methyl-4-[(2-methyl-5-phenyl-1,3-thiazol-4-yl)carbonyl]-1-(2-phenylpyrimidin-4-yl)-1,4-diazepan,
(2S)-2-Methyl-1-[5-methyl-2-(2H-1,2,3-triazol-2-yl)benzoyl]-4-(6-phenylpyrazin-2-yl)-1,4-diazepan,
4-Chlor-2-{(3S)-3-methyl-4-[5-methyl-2-(2H-1,2,3-triazol-2-yl)benzoyl]-1,4-diazepan-1-yl}-1,3-benzothiazol,
2-{(3S)-4-[2-(1H-Imidazol-1-yl)benzoyl]-3-methyl-1,4-diazepan-1-yl} chinazolin,
2-[(3S)-4-(2,6-Dimethoxybenzoyl)-3-methyl-1,4-diazepan-1-yl]chinazolin,
2-{(3S)-3-Methyl-4-[2-(1H-pyrrol-1-yl)benzoyl]-1,4-diazepan-1-yl chinazolin,
N-Ethyl-2-{[(2S)-2-methyl-4-chinazolin-2-yl-1,4-diazepan-1-yl]carbonyl}anilin,
2-{4-[2-(2H-1,2,3-Triazol-2-yl)benzoyl]-4,8-diazaspiro[2.6]non-8-yl}chinazolin, 8-(4-Phenylpyrimidin-2-yl)-4-(2-(2H-1,2,3-triazol-2-yl)benzoyl]-4,8-diazaspiro[2.6]nonan,
oder ein pharmazeutisch annehmbares Salz davon.

13. Eine pharmazeutische Zusammensetzung, die einen inerten Träger und eine Verbindung gemäß einem vorhergehenden Anspruch oder ein pharmazeutisch annehmbares Salz davon umfasst.

14. Eine Verbindung gemäß einem der Ansprüche 1-12 zur Verwendung bei der Behandlung oder Prävention einer Schlafstörung.

15. Eine Verbindung gemäß einem der Ansprüche 1-12 zur Verwendung bei der Behandlung oder Steuerung von Adipositas bei einem Patienten, der ein Säuger ist und der eine solche Behandlung benötigt.

## Revendications

1. Composé de la formule I: 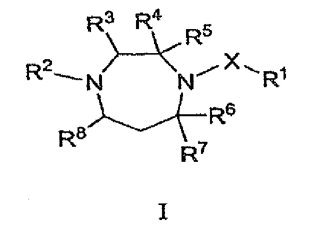 où:
X est sélectionné parmi -SO₂-, -(C=O)- et -(C=S)-;
R¹ est sélectionné parmi le groupe consistant en:
(1) phényle, où le phényle est substitué par R^{1a}, R^{1b} et R^{1c} et
(2) naphtyle, où le naphtyle est substitué par R^{1a}, R^{1b} et R^{1c};
(3) hétéroaryle, où l'hétéroaryle est substitué par R^{1a}, R^{1b} et R^{1c};
R² est hétéroaryle, où l'hétéroaryle est substitué par R^{2a}, R^{2b} et R^{2c};
R^{1a}, R^{1b}, R^{1c}, R^{2a}, R^{2b} et R^{2c} sont sélectionnés indépendamment parmi le groupe consistant en:
(1) hydrogène,
(2) halogène,
(3) hydroxyle,
(4) -(C=O)ₘ-Oₙ-alkyle C₁₋₆, où m est 0 ou 1, n est 0 ou 1 (où si m est 0 ou n est 0, une liaison est présente) et où l'alkyle est non substitué ou substitué par un ou plusieurs substituants sélectionnés de R¹³,
(5) -(C=O)ₘ-Oₙ-cycloalkyle C₃₋₆, où le cycloalkyle est non substitué ou substitué par un ou plusieurs substituants sélectionnés de R¹³,
(6) -(C=O)ₘ-alcényle C₂₋₄, où l'alcényle est non substitué ou substitué par un ou plusieurs substituants sélectionnés de R¹³,
(7) -(C=O)ₘ-alcynyle C₂₋₄, où l'alcynyle est non substitué ou substitué par un ou plusieurs substituants sélectionnés de R¹³,
(8) -(C=O)ₘ-Oₙ-phényle ou -(C=O)ₘ-Oₙ-naphtyle, où le phényle ou le naphtyle est non substitué ou substitué par un ou plusieurs substituants sélectionnés de R¹³,
(9) -(C=O)ₘ-Oₙ-hétérocycle, où l'hétérocycle est non substitué ou substitué par un ou plusieurs substituants sélectionnés de R¹³,
(10) -(C=O)ₘ-NR¹⁰R¹¹, où R¹⁰ et R¹¹ sont sélectionnés indépendamment parmi le groupe consistant en:
(a) hydrogène,
(b) alkyle C₁₋₆, qui est non substitué ou substitué par un ou plusieurs substituants sélectionnés de R¹³,
(c) alcényle C₃₋₆, qui est non substitué ou substitué par un ou plusieurs substituants sélectionnés de R¹³,
(d) cycloalkyle, qui est non substitué ou substitué par un ou plusieurs substituants sélectionnés de R¹³,
(e) phényle, qui est non substitué ou substitué par un ou plusieurs substituants sélectionnés de R¹³ et
(f) hétérocycle, qui est non substitué ou substitué par un ou plusieurs substituants sélectionnés de R¹³,
(11) -S(O)₂-NR¹⁰R¹¹,
(12) -S(O)_{q}-R¹² où q est 0, 1 ou 2 et où R¹² est sélectionné parmi les définitions de R¹⁰ et de R¹¹,
(13) -CO₂H,
(14) -CN,
(15) -NO₂ et
(16) -B(OH)₂;
R³, R⁴, R⁵, R⁶, R⁷, et R⁸ sont sélectionnés indépendamment parmi le groupe consistant en:
(1) hydrogène,
(2) -alkyle C₁₋₆, où l'alkyle est non substitué ou substitué par un ou plusieurs substituants sélectionnés de R¹³,
(3) -O-alkyle C₁₋₆, où l'alkyle est non substitué ou substitué par un ou plusieurs substituants sélectionnés de R¹³ et
(4) -phényle, où le phényle est non substitué ou substitué par un ou plusieurs substituants sélectionnés de R¹³,
ou bien R⁴ et R⁵ ou R⁶ et R⁷ sont pris ensemble pour former un cycloalkyle C₃₋₆, où le cycloalkyle est non substitué ou substitué par un ou plusieurs substituants sélectionnés de R¹⁴,
à condition qu'au moins l'un d'entre R³, R⁴, R⁵, R⁶, R⁷ ou R⁸ soit autre que hydrogène;
R¹³ est sélectionné parmi le groupe consistant en:
(1) halogène,
(2) hydroxyle,
(3) -(C=O)ₘ-Oₙ-alkyle C₁₋₆, où l'alkyl est non substitué ou substitué par un ou plusieurs substituants sélectionnés de R¹⁴,
(4) -Oₙ-perfluoroalkyl-(C₁₋₃),
(5) -(C=O)ₘ-Oₙ-cycloalkyle C₃₋₆, où le cycloalkyle est non substitué ou substitué par un ou plusieurs substituants sélectionnés de R¹⁴,
(6) -(C=O)ₘ-alcényle C₂₋₄, où l'alcényle est non substitué ou substitué par un ou plusieurs substituants sélectionnés de R¹⁴,
(7) -(C=O)ₘ-Oₙ-phényle ou -(C=O)ₘ-Oₙ-naphtyle, où le phényle ou le naphtyle est non substitué ou substitué par un ou plusieurs substituants sélectionnés de R¹⁴,
(8) -(C=O)ₘ-Oₙ-hétérocycle, où l'hétérocycle est non substitué ou substitué par un ou plusieurs substituants sélectionnés de R¹⁴,
(9) -(C=O)ₘ-NR¹⁰R¹¹,
(10) -S(O)₂-NR¹⁰R¹¹,
(11) -S(O)_{q}-R¹²,
(12) -CO₂H,
(13) -CN et
(14) -NO₂;
R¹⁴ est sélectionné parmi le groupe consistant en:
(1) hydroxyle,
(2) halogène,
(3) alkyle C₁₋₆
(4) -cycloalkyle C₃₋₆,
(5) -O-alkyle C₁₋₆,
(6) -O(C=O)-alkyle C₁₋₆,
(7) -NH-alkyle C₁₋₆,
(8) phényle,
(9) hétérocycle,
(10) -CO₂H et
(11) -CN;
ou un sel pharmaceutiquement acceptable de celui-ci.

2. Composé de la revendication 1 de la formule Ia: 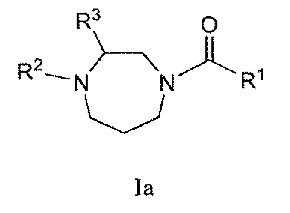 ou de la formule Ib: 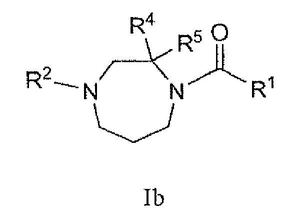 ou de la formule Ic: 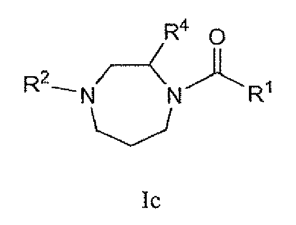 ou de la formule Ic': 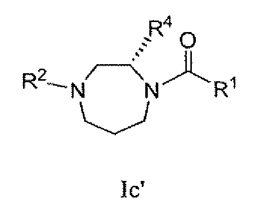 ou de la formule Id: 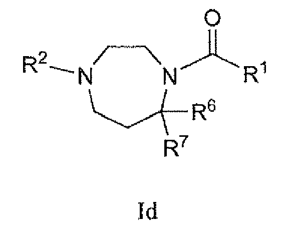 ou de la formule Ie: 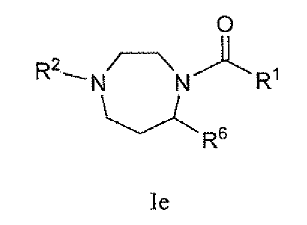 ou de la formule If: 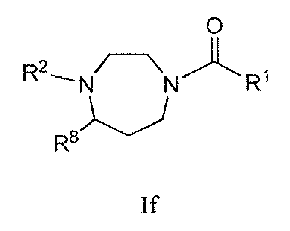 ou un sel pharmaceutiquement acceptable de celui-ci.

3. Composé de la revendication 1, dans lequel X est -(C=O)-.

4. Composé de la revendication 1, dans lequel R¹ est phényle qui est non substitué ou substitué par un ou plusieurs d'entre:
(1) halogène,
(2) hydroxyle,
(3) -Oₙ-alkyle C₁₋₆, où n est 0 ou 1 (où si n est 0, une liaison est présente) et où l'alkyle est non substitué ou substitué par un ou plusieurs substituants sélectionnés de R¹³,
(4) -Oₙ-phényle, où le phényle est non substitué ou substitué par un ou plusieurs substituants sélectionnés de R¹³,
(5) -hétérocycle, où l'hétérocycle est non substitué ou substitué par un ou plusieurs substituants sélectionnés de R¹³,
(6) -NR¹⁰R¹¹, où R¹⁰ et R¹¹ sont sélectionnés indépendamment parmi le groupe consistant en:
(a) hydrogène,
(b) alkyle C₁₋₆, qui est non substitué ou substitué par un ou plusieurs substituants de R¹³,
(7) -S(O)₂-NR¹⁰R¹¹,
(8) -CO₂H,
(9) -CN,
(10) -NO₂ et
(11) -B(OH)₂.

5. Composé de la revendication 4, dans lequel R¹ est sélectionné parmi le groupe consistant en:
(1) phényle,
(2) biphényle,
(3) 2,6-diméthoxyphényle,
(4) 2,4-dichlorophényle,
(5) 2,6-dichlorophényle,
(6) 2,3-difluorophényle,
(7) 2,4-difluorophényle,
(8) 2,6-difluorophényle,
(9) 2-méthoxy-4-méthyl-phényle,
(10) 3-méthoxy-biphényle,
(11) 3-méthyl-biphényle et
(12) 5-méthyl-2-triazolyl-phényle.

6. Composé de la revendication 1, dans lequel R² est sélectionné parmi le groupe consistant en:
(1) benzimidazolyle,
(2) benzothiazolyle,
(3) benzoxazolyle,
(4) quinazolinyle,
(5) quinolinyle et
(6) thiadiazolyle,
lequel est non substitué ou substitué par halogène, hydroxyle, alkyle C₁₋₆, -O-alkyle C₁₋₆ ou phényle.

7. Composé de la revendication 6, dans lequel R² est sélectionné parmi le groupe consistant en:
(1) benzimidazol-2-yle,
(2) 1,3-benzothiazol-2-yle,
(3) 1,3-benzoxazol-2-yle,
(4) 2-quinazolinyle,
(5) 1-quinolin-2-yle et
(6) 1,2,4-thiadiazol-5-yle,
lequel est non substitué ou substitué par méthyle, fluoro, chloro ou phényle.

8. Composé de la revendication 1, dans lequel R³, R⁴, R⁵, R⁶, R⁷ et R⁸ sont sélectionnés indépendamment parmi le groupe consistant en:
(1) hydrogène et
(2) -alkyle C₁₋₆, où l'alkyle est non substitué ou substitué par un ou plusieurs substituants sélectionnés de R¹³,
ou bien R⁴ et R⁵ ou R⁶ et R⁷ sont pris ensemble pour former un cycloalkyle C₃₋₆, où le cycloalkyle est non substitué ou substitué par un ou plusieurs substituants sélectionnés de R¹⁴,
à condition que l'un au moins d'entre R³, R⁴, R⁵, R⁶, R⁷ ou R⁸ soit autre que hydrogène.

9. Composé de la revendication 1, dans lequel l'un d'entre R³ et R⁴ est -alkyle C₁₋₆ et les autres de R³ à R⁸ sont un hydrogène.

10. Composé de la revendication 1, dans lequel l'un d'entre R⁶ et R⁸ est -alkyle C₁₋₆ et les autres de R³ à R⁸ sont un hydrogène.

11. Composé de la revendication 1, dans lequel R⁴ et R⁵ sont pris ensemble pour former un cycloalkyle C₃₋₆, ou bien R⁶ et R⁷ sont pris ensemble pour former un cycloalkyle C₃₋₆ et les autres de R³ à R⁸ sont un hydrogène.

12. Composé de la revendication 1, lequel est sélectionné parmi le groupe consistant en:
2-{5-méthyl-4-[5-méthyl-2-(2H-1,2,3-triazol-2-yl)benzoyl]-1,4-diazépan-1-yl}-1,3-benzoxazole;
2-{5-méthyl-4-[2-(2H-1,2,3-triazol-2-yl)benzoyl]-1,4-diazépan-1-yl}quinazoline;
2-{3-méthyl-4-[5-méthyl-2-(2H-1,2,3-triazol-2-yl)benzoyl]-1,4-diazépan-1-yl} - 1,3-benzoxazole;
(S)-2- {3-méthyl-4-[5-méthyl-2-(2H-1,2,3-triazol-2-yl)benzoyl]-1,4-diazépan-1-yl}-1,3-benzoxazole;
2-{2-méthyl-4-[5-méthyl-2-(2H-1,2,3-triazol-2-yl)benzoyl]-1,4-diazépan-1-yl} - 1,3-benzoxazole;
2-{7-méthyl-4-[5-méthyl-2-(2H-1,2,3-triazol-2-yl)benzoyl]-1,4-diazépan-1-yl}-1,3-benzoxazole;
5-méthyl-1-(4-phénylpyrimidin-2-yl)-4-[2-(2H-1,2,3-triazol-2-yl)benzoyl]-1,4-diazépane;
2-{5-méthyl-4-[2-(2H-1,2,3-triazol-2-yl)benzoyl]-1,4-diazépan-1-yl}quinoline;
2-{5-méthyl-4-[(2-méthyl-5-phényl-1,3-thiazol-4-yl)carbonyl]-1,4-diazépan-1-yl}quinazoline;
5-méthyl-4-[(2-méthyl-5-phényl-1,3-thiazol-4-yl)carbonyl]-1-(2-phénylpyrimidin-4-yl)-1,4-diazépane;
(2S)-2-méthyl-1-[5-méthyl-2-(2H-1,2,3-triazol-2-yl)benzoyl]-4-(6-phénylpyrazin-2-yl)-1,4-diazépane;
4-chloro-2-{(3S)-3-méthyl-4-[5-méthyl-2-(2H-1,2,3-triazol-2-yl)benzoyl]-1,4-diazépan-1-yl}-1,3-benzothiazole;
2- {(3S)-4-[2-(1H-imidazol-1-yl)benzoyl]-3-méthyl-1,4-diazépan-1-yl}quinazoline;
2-[(3S)-4-(2,6-diméthoxybenzoyl)-3-méthyl-1,4-diazépan-1-yl} quinazoline;
2-{(3S)-3-méthyl-4-[2-(1H-pyrrol-1-yl)benzoyl]-1,4-diazépan-1-yl}quinazoline;
N-éthyl-2-{[(2S)-2-méthyl-4-quinazolin-2-yl-1,4-diazépan-1-yl] carbonyL}aniline;
2-{4-[2-(2H-1,2,3-triazol-2-yl)benzoyl]-4,8-diazaspiro[2.6]non-8-yl}quinazoline;
8-(4-phénylpyrimidin-2-yl)-4-[2-(2H-1,2,3-triazol-2-yl)benzoyl]-4,8-diazaspiro[2.6]nonane;
ou un sel pharmaceutiquement acceptable de celui-ci.

13. Composition pharmaceutique qui comprend un excipient inerte et un composé de l'une quelconque des revendications précédentes ou un sel pharmaceutiquement acceptable de celui-ci.

14. Composé selon l'une quelconque des revendications 1-12 à utiliser dans le traitement ou la prévention d'un trouble du sommeil.

15. Composé selon l'une quelconque des revendications 1-12 à utiliser dans le traitement ou le contrôle de l'obésité chez un patient mammalien en ayant besoin.
